# EUROPÄISCHE PATENTSCHRIFT

(11) **EP 3 880 729 B1**
(45) Veröffentlichungstag und Bekanntmachung des Hinweises auf die Patenterteilung: **27.09.2023**
(21) Anmeldenummer: 19798111.1
(22) Anmeldetag: 11.11.2019
(51) Int. Cl.: C08G 18/48, C08G 18/75, C08G 18/76, C08G 18/12, C08G 18/16, C08G 18/24, C08G 18/28, C08G 18/32

(54) **THIXOTROPIERMITTEL FÜR HÄRTBARE ZUSAMMENSETZUNGEN**
THIXOTROPIC AGENTS FOR CURABLE COMPOSITIONS
AGENT THIXOTROPE POUR COMPOSITIONS DURCISSABLES

(30) Priorität: 14.11.2018 EP 18206318
(43) Veröffentlichungstag der Anmeldung: 22.09.2021
(73) Patentinhaber: Sika Technology AG, 6340 Baar (CH)
(72) Erfinder: KRAMER, Andreas, 8008 Zürich (CH); BURCKHARDT, Urs, 8049 Zürich (CH); SCHLUMPF, Michael, 8143 Stallikon (CH); KONSTANZER, Martin, 5000 Aarau (CH)
(74) Vertreter: Sika Patent Attorneys
(86) Internationale Anmeldenummer: PCT/EP2019/080856
(87) Internationale Veröffentlichungsnummer: WO 2020/099314

(56) Entgegenhaltungen:
- US-A1- 2002 007 003
- US-A1- 2007 066 721

## Beschreibung

### Technisches Gebiet

Die Erfindung betrifft Thixotropiermittel und damit verdickte härtbare Zusammensetzungen, insbesondere elastische Klebstoffe, Dichtstoffe oder Beschichtungen.

### Stand der Technik

In härtbaren Zusammensetzungen auf Basis von Polyurethanpolymeren oder silanmodifizierten Polymeren (SMP) werden oft sogenannte Thixotropiermittel eingesetzt, um deren Fliessgrenze und somit Standfestigkeit zu erhöhen und damit die Verarbeitbarkeit der Produkte zu verbessern. Vor allem für pastöse Kleb- und Dichtstoffe ist eine hohe Standfestigkeit bei nicht zu hoher Auspresskraft und kurzem Fadenzug beim Absetzen des Auftragswerkzeugs wichtig. Als Thixotropiermittel besonders wirksam und kostengünstig sind feinteilige Dispersionen von Harnstoffverbindungen, insbesondere Umsetzungsprodukte von Diisocyanaten mit niedermolekularen primären Aminen, welche in einem flüssigen Trägermaterial hergestellt werden und homogene, streichfähige Pasten darstellen. Ihr Gehalt an Harnstoffverbindungen beträgt typischerweise nur etwa zwanzig Prozent, wodurch es für eine gute Thixotropierung relativ viel Paste braucht und dementsprechend viel Trägermaterial in das zu verdickende Produkt eingetragen wird. Dies kann zu Problemen mit Migrationseffekten führen, wie insbesondere Ausschwitzen (Bleeding), Flecken- oder Hofbildung (Staining), insbesondere bei porösen Substraten, Aufweichung, Rissbildung, Verfärbung oder Verlust der Haftung des Substrats oder einer darüber applizierten Lack- oder Farbschicht. Bei Kontakt mit Lösemitteln oder Treibstoffen kann es zur Quellung (Swelling) kommen, gefolgt von der Auswaschung des Trägermaterials, was zu Rissbildung und Versprödung der Polymermatrix führen kann. Zudem kann das Trägermaterial beim Erwärmen verdampfen und damit zu Ausgasungsverlusten (Fogging) führen. Als Trägermaterial für solche Thixotropierpasten eignen sich insbesondere die üblichen in Kleb- und Dichtstoffen zur Einstellung der Härte und Elastizität eingesetzten Weichmacher. Besonders geeignete Weichmacher sind höhermolekulare Phthalate, insbesondere Diisodecylphthalat (DIDP) oder Diisononylphthalat (DINP). Sie ermöglichen sehr feinteilige Pasten, welche gut verdicken, und verursachen nur wenig Migrationseffekte. Bei Kontakt mit Laugen, beispielsweise bei Applikation auf frischem Beton oder durch basische Formulierungsbestandteile, können sie aber verseifen und dabei Versprödung oder Depolymerisation verursachen, oft im Verbund mit Geruchsimmissionen ausgehend von den freigesetzten Fettalkoholen. Ausserdem erhöhen sie die Steifigkeit bzw. den Elastizitätsmodul eines Kleb- und Dichtstoffs in der Kälte im Vergleich zu Raumtemperatur überproportional. Und schliesslich besteht wegen weitverbreiteter Skepsis gegenüber Phthalaten zudem ein Wunsch nach phthalatfreien Produkten. Alternativen zu den Phthalat-Weichmachern sind insbesondere Cyclohexandicarbonsäureester, wie beispielsweise Diisononyl-1,2-cyclohexandicarboxylat (DINCH), oder Adipate, wie beispielsweise Dioctyladipat bzw. Bis(2-ethylhexyl)adipat (DOA). Diese zeigen aber eine deutlich höhere Neigung zu Migrationseffekten und setzen beim Verseifen ebenfalls geruchsintensive Fettalkohole frei. Sie sind daher als Trägermaterial weniger geeignet.

In EP 1 152 019 werden Thixotropiermittel auf Basis von Harnstoffverbindungen beschrieben, welche als Trägermaterial anstelle von Weichmachern Polyole einsetzen. Die damit erhaltenen Pasten sind hochviskos und schlecht handhabbar, insbesondere schlecht pumpbar, und ihre Wirkung auf die Standfestigkeit ist deutlich geringer als bei den auf herkömmlichen Weichmachern basierenden Pasten. Da die Polyole mit Isocyanatgruppen-haltigen Zusammensetzungen reagieren, verursachen sie zwar keine Migrationseffekte, führen aber zu einer starken Erhöhung der Viskosität, was für eine gute Verarbeitbarkeit und den Fadenzug beim Absetzen des Applikationswerkzeugs der damit verdickten Produkte nachteilig ist.

In EP19798111 werden ähnliche urethangruppenhaltige Thixotropiermittel für Epoxidharze beschrieben, welche die selben nachteiligen Eigenschaften besitzen.

### Darstellung der Erfindung

Aufgabe der vorliegenden Erfindung ist es daher, ein Thixotropiermittel auf Basis von Harnstoffverbindungen für härtbare Zusammensetzungen auf Polyurethan- oder SMP-Basis zur Erhöhung der Fliessgrenze bereitzustellen, welches frei von Phthalaten ist und die Nachteile des Standes der Technik in Bezug auf Viskositätserhöhung und Neigung zu Migration oder Fogging überwindet.

Die Aufgabe wird mit dem Thixotropiermittel wie in Anspruch 1 beschrieben gelöst. Das Thixotropiermittel ist aus gut erhältlichen Grundstoffen in einem einfachen Verfahren herstellbar, unreaktiv gegenüber Isocyanat- und Silangruppen und von hervorragender Qualität. Im Vergleich mit herkömmlichen Thixotropiermitteln auf Basis von Harnstoffverbindungen erhöht das erfindungsgemässe Thixotropiermittel die Fliessgrenze von damit verdickten Zusammensetzungen in gleichem oder sogar höherem Mass, verursacht keine stärkere Neigung zu Migrationseffekten und setzt bei Verseifung bzw. Hydrolyse des Trägermaterials keine geruchsintensiven, flüchtigen Spaltprodukte frei. Überraschenderweise ist das erfindungsgemässe Thixotropiermittel bei gleicher Konzentration, Herstellung und Standfestigkeit sogar leichter auspressbar (geringere Auspresskraft durch eine Düse von einigen Millimetern), wodurch damit verdickte Zusammensetzungen besonders gut verarbeitbar sind. Zudem zeigen die ausgehärteten Zusammensetzungen eine besonders gute Flexibilität in der Kälte.

Das erfindungsgemässe Thixotropiermittel zeigt schon bei niedriger Dosierung eine hohe Thixotropierwirkung, also Erhöhung der Fliessgrenze bzw. Standfestigkeit, und führt weder zu verstärktem Auftreten von durch Weichmacher-Migration bedingten Effekten wie Bleeding oder Staining noch zu erhöhtem Fogging. Es eignet sich insbesondere für die Verwendung in Klebstoffen, Dichtstoffen oder Beschichtungen auf Polyurethan- oder SMP-Basis, welche bevorzugt phthalatfrei sind.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Thixotropiermittel zur Erhöhung der Fliessgrenze einer härtbaren Zusammensetzung, wobei das Thixotropiermittel (i) mindestens eine Harnstoffverbindung aus der Umsetzung von mindestens einem Isocyanat mit mindestens einem Amin und (ii) mindestens einen Polyether mit blockierten Hydroxylgruppen umfasst, welcher frei von Urethangruppen ist.

Als "blockierte Hydroxylgruppe" wird eine durch chemische Reaktion zu einer gegenüber Isocyanatgruppen nicht reaktiven Gruppe umgesetzte Hydroxylgruppe bezeichnet.

Als "Polyether" wird ein Molekül bzw. eine Gruppe von oligomeren und/oder polymeren Molekülen bezeichnet, welche mehrheitlich aus Alkylenoxy-Repetiereinheiten bestehen.

Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.

Als "primäres Monoamin" wird eine Verbindung mit einer primären Aminogruppe bezeichnet, die keine weiteren Aminogruppen aufweist.

Als "härtbare Zusammensetzung" wird eine Zusammensetzung enthaltend polymerisierbare Makromoleküle bezeichnet, die durch Vernetzungsreaktionen ihrer Reaktivgruppen aushärten bzw. einen Zustand erhöhter mechanischer Festigkeit erlangen kann.

Als "Silangruppe" wird eine an einen organischen Rest gebundene Silylgruppe mit einem bis drei, insbesondere zwei oder drei, hydrolysierbaren Alkoxy-Resten am Silicium-Atom bezeichnet.

Als "Silan" werden sowohl Organoalkoxysilane, welche eine bis drei organische Substituenten an jeder Silangruppe tragen, als auch Tetraalkoxysilane bezeichnet. Silane, die an einem organischen Rest zusätzlich zur Silangruppe eine oder mehrere Hydroxyl-, Isocyanato-, Amino- bzw. Mercaptogruppen tragen, werden als "Hydroxysilan", "Isocyanatosilan", "Aminosilan" bzw. "Mercaptosilan" bezeichnet.

Als "Weichmacher" werden schwerflüchtige, die Viskosität eines Polymers senkende Substanzen bezeichnet, welche im Polymer nicht chemisch eingebunden sind und auf dieses eine weichmachende Wirkung ausüben.

Mit "Poly" beginnende Substanznamen wie Polyamin, Polyol oder Polyisocyanat bezeichnen Substanzen, die formal zwei oder mehr der in ihrem Namen vorkommenden funktionellen Gruppen pro Molekül enthalten.

Als "Molekulargewicht" wird die molare Masse (in Gramm pro Mol) eines Moleküls oder eines Molekül-Rests bezeichnet. Als "mittleres Molekulargewicht" wird das Zahlenmittel des Molekulargewichts Mₙ einer polydispersen Mischung von oligomeren oder polymeren Molekülen oder Molekül-Resten bezeichnet. Es wird üblicherweise mittels Gelpermeationschromatographie (GPC) gegen Polystyrol als Standard bestimmt.

Als "lagerstabil" oder "lagerfähig" wird eine härtbare Zusammensetzung bezeichnet, die bei Raumtemperatur in einem geeigneten Gebinde während längerer Zeit, typischerweise während mindestens 3 Monaten bis zu 6 Monaten und mehr, aufbewahrt werden kann, ohne dass sie sich in ihren Anwendungs- oder Gebrauchseigenschaften durch die Lagerung in einem für ihren Gebrauch relevanten Ausmass verändert.

Als "Raumtemperatur" wird eine Temperatur von 23°C bezeichnet.

Bevorzugt umfasst das Thixotropiermittel eine Harnstoffverbindung aus der Umsetzung von mindestens einem Diisocyanat mit mindestens einem primären Monoamin. Dabei werden die Aminogruppen in Bezug auf die Isocyanatgruppen bevorzugt stöchiometrisch eingesetzt.

Als Diisocyanat bevorzugt ist 1,6-Hexamethylendiisocyanat, 1-Isocyanato-3,3,5-trimethyl-5-isocyanatomethylcyclohexan, Perhydro-4,4'-diphenylmethandiisocyanat, 1,3-Phenylendiisocyanat, 1,4-Phenylendiisocyanat, 2,4-Toluylendiisocyanat oder Gemische davon mit 2,6-Toluylendiisocyanat (TDI), oder 4,4'-Diphenylmethandiisocyanat, gegebenenfalls mit Anteilen von 2,4'- und/oder 2,2'-Diphenylmethandiisocyanat (MDI).

Besonders bevorzugt ist 2,4-Toluylendiisocyanat oder 4,4'-Diphenylmethandiisocyanat.

Am meisten bevorzugt ist 4,4'-Diphenylmethandiisocyanat.

Damit werden Thixotropiermittel mit besonders guter Wirkung erhalten.

Als primäres Monoamin bevorzugt ist ein Alkylamin, insbesondere ein Alkylamin mit 1 bis 12 C-Atomen. Besonders bevorzugt ist ein Alkylamin mit 4 bis 6 C-Atomen, insbesondere Butylamin, Hexylamin oder Cyclohexylamin.

Am meisten bevorzugt ist n-Butylamin. Damit werden Thixotropiermittel mit besonders guter Wirkung erhalten.

Besonders bevorzugte Harnstoffverbindungen sind Umsetzungsprodukte von 4,4'-Diphenylmethandiisocyanat und n-Butylamin.

Bevorzugt weist die Harnstoffverbindung die Formel (I) auf, 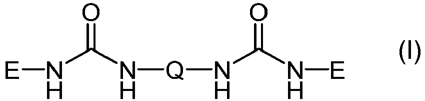 wobei
E für einen Alkyl- oder Cycloalkyl-Rest mit 1 bis 12 C-Atomen steht, und
Q für einen zweiwertigen Kohlenwasserstoff-Rest mit 6 bis 15 C-Atomen steht.

Bevorzugt steht E für einen Alkyl- oder Cycloalkyl-Rest mit 4 bis 6 C-Atomen, insbesondere für Butyl oder Hexyl oder Cyclohexyl.

Am meisten bevorzugt steht E für Butyl, insbesodere für n-Butyl.

Bevorzugt steht Q für 1,6-Hexylen, (1,5,5-Trimethylcyclohexan-1-yl)methan-1,3, Methylenbis(cyclohexan-4-yl), 1,3-Phenylen, 1,4-Phenylen, 4(2)-Methyl-1,3-phenylen oder Methylenbis(phen-4-yl).

Besonders bevorzugt steht Q für 4-Methyl-1,3-phenylen oder Methylenbis(phen-4-yl).

Am meisten bevorzugt steht Q für Methylenbis(phen-4-yl).

Bevorzugt wird das Thixotropiermittel hergestellt, indem die Umsetzung zwischen dem Isocyanat und dem Amin im Polyether mit blockierten Hydroxylgruppen durchgeführt wird.

Bevorzugt wird die Umsetzung bei einer Temperatur im Bereich von 0 bis 100°C, insbesondere 10 bis 80°C, durchgeführt.

Bei der Herstellung des Thixotropiermittels ist es vorteilhaft, eine Methode zu verwenden, bei welcher die eingesetzten Reaktanden und die Reaktionsmischung so gerührt und/oder gefördert werden, dass die entstehenden Harnstoffverbindungen in möglichst feinteiliger Form ausfallen, damit eine möglichst homogene Paste mit hoher Thixotropierwirkung entsteht.

Der Polyether mit blockierten Hydroxylgruppen kann zusammen mit dem Isocyanat vorgelegt und das Amin unter gutem Rühren langsam zugegeben werden. Weiterhin kann der Polyether mit blockierten Hydroxylgruppen zusammen mit dem Amin vorgelegt und das Isocyanat unter gutem Rühren langsam zugegeben werden.

Weiterhin kann das Amin und/oder das Isocyanat mit etwas Polyether mit blockierten Hydroxylgruppen verdünnt werden, bevor es eingesetzt wird. Bevorzugt erfolgt die Herstellung der Harnstoffverbindungen in einem kontinuierlichen Prozess, wobei das Amin und/oder das Isocyanat gegebenenfalls in einer mit Polyether mit blockierten Hydroxylgruppen verdünnten Form eingesetzt werden.

Bevorzugt wird die Herstellung unter Ausschluss von Feuchtigkeit durchgeführt. Bevorzugt sind bei der Herstellung keine weiteren Additive vorhanden, insbesondere keine zugefügten Lösemittel und keine zugefügten Katalysatoren.

Bevorzugt liegt das Thixotropiermittel in Form einer bei Raumtemperatur standfesten, streichfähigen Paste vor.

Bevorzugt enthält das Thixotropiermittel 5 bis 25 Gewichts-% Harnstoffverbindungen und 50 bis 95 Gewichts-% Polyether mit blockierten Hydroxylgruppen. Besonders bevorzugt enthält das Thixotropiermittel 10 bis 25 Gewichts-% Harnstoffverbindungen und 75 bis 90 Gewichts-% Polyether mit blockierten Hydroxylgruppen.

Der Polyether mit blockierten Hydroxylgruppen ist im Wesentlichen frei von nicht blockierten Hydroxylgruppen. Dabei bedeutet "im Wesentlichen frei von", dass 95 %, bevorzugt 99 %, insbesondere 99.9%, am meisten bevorzugt 100 %, der vorhandenen Hydroxylgruppen blockiert sind.

Der Polyether mit blockierten Hydroxylgruppen ist bevorzugt frei von Reaktivgruppen, welche mit Feuchtigkeit oder mit in der Zusammensetzung vorhandenen Inhaltsstoffen Vernetzungsreaktionen eingehen. Er ist also inbesondere frei von Isocyanatgruppen und Silangruppen.

Der Polyether mit blockierten Hydroxylgruppen ist insbesondere bei Raumtemperatur flüssig.

Bevorzugt weist der Polyether mit blockierten Hydroxylgruppen eine Viskosität bei 20°C im Bereich von 30 bis 500 mPa·s, insbesondere 50 bis 250 mPa·s, auf. Dabei wird die Viskosität bestimmt mit einem Kegel-Platten-Viskosimeter mit Kegeldurchmesser 25 mm, Kegelwinkel 1°, Kegelspitze-Platten-Abstand 0.05 mm bei einer Scherrate von 10 s⁻¹. Damit werden gut handhabbare Pasten mit hoher Thixotropierwirkung erhalten.

Bevorzugt weist der Polyether im Mittel 1 bis 3 blockierte Hydroxylgruppen pro Molekül auf, insbesondere 1 oder 2.

Bevorzugt sind die blockierten Hydroxylgruppen ausgewählt aus der Gruppe bestehend aus Acetal-, Ester-, Acetoester- und Carbonatgruppen.

Bevorzugt weisen diese Acetal-, Ester-, Acetoester- oder Carbonatgruppen 1 bis 15 C-Atome auf.

Besonders bevorzugt ist eine Estergruppe, insbesondere eine Estergruppe mit 1 bis 8 C-Atomen.

Am meisten bevorzugt ist eine Acetatgruppe. Ein Polyether mit blockierten Hydroxylgruppen in Form von Acetatgruppen ist besonders niedrigviskos, besonders einfach zugänglich und besonders preisgünstig.

Bevorzugt als Acetalgruppe ist eine 1-(Isobutoxy)ethoxy- oder eine Tetrahydropyran-2-oxy- oder eine Tetrahydrofuran-2-oxy-Gruppe, insbesondere eine 1-(Isobutoxy)ethoxy-Gruppe.

Bevorzugt als Acetoestergruppe ist eine Acetoacetatgruppe.

Bevorzugt als Carbonatgruppe ist eine Methylcarbonatgruppe.

Diese sind niedrigviskos und aus preisgünstigen Ausgangsstoffen zugänglich.

Bevorzugt weist der Polyether mit blockierten Hydroxylgruppen als Repetiereinheiten 1,2-Ethylenoxy-, 1,2-Propylenoxy-, 1,3-Propylenoxy-, 1,2-Butylenoxy- oder 1,4-Butylenoxy-Gruppen, insbesondere 1,2-Propylenoxy-Gruppen, auf.

Bevorzugt bestehen 70 bis 100 Gewichts-%, insbesondere 80 bis 100 Gewichts-%, der Repetiereinheiten aus 1,2-Propylenoxy-Gruppen und 0 bis 30 Gewichts-%, insbesondere 0 bis 20 Gewichts-%, der Repetiereinheiten aus 1,2-Ethylenoxy-Gruppen.

Besonders bevorzugt bestehen die Repetiereinheiten zu 100 % aus 1,2-Propylenoxy-Gruppen.

Solche Polyether sind einfach verfügbar und hydrophob und ermöglichen thixotropierte härtbare Zusammensetzungen mit geringer Wasseraufnahme und guter Beständ ig keit.

Bevorzugt weist der Polyether mit blockierten Hydroxylgruppen ein mittleres Molekulargewicht Mₙ im Bereich von 600 bis 2'500 g/mol, mehr bevorzugt 700 bis 2'000 g/mol, insbesondere 800 bis 1'500 g/mol, auf, bestimmt mittels Gelpermeationschromatographie (GPC) gegen Polystyrol als Standard mit Tetrahydrofuran als mobiler Phase, Brechungsindex-Detektor und Auswertung ab 200 g/mol. Damit werden gut handhabbare Pasten mit hoher Thixotropierwirkung erhalten, welche in härtbaren Zusammensetzungen keine Probleme mit Emission oder Geruch verursachen.

Bevorzugt ist der Polyether mit blockierten Hydroxylgruppen abgeleitet von mindestens einem hydroxyfunktionellen Polyether ausgewählt aus der Gruppe bestehend aus
- Alkohol-, insbesondere n-Butanol-, gestarteten Polyoxypropylen-Monolen mit einer OH-Zahl im Bereich von 25 bis 90 mg KOH/g, bevorzugt 50 bis 80 mg KOH/g,
- Polyoxypropylen-Diolen mit einer OH-Zahl im Bereich von 45 bis 155 mg KOH/g, bevorzugt 56 bis 125 mg KOH/g, und
- Trimethylolpropan- oder insbesondere Glycerin-gestarteten, gegebenenfalls Ethylenoxid-terminierten, Polyoxypropylen-Triolen mit einer mittleren OH-Funktionalität im Bereich von 2.2 bis 3 und einer OH-Zahl im Bereich von 90 bis 230 mg KOH/g.

Davon bevorzugt sind Alkohol-, insbesondere n-Butanol-, gestartete Polyoxypropylen-Monole oder Polyoxypropylen-Diole.

Besonders bevorzugt sind Polyoxypropylen-Diole. Diese sind besonders preisgünstig.

Der Polyether mit blockierten Hydroxylgruppen wird insbesondere erhalten durch Umsetzung von mindestens einem hydroxyfunktionellen Polyether mit mindestens einem geeigneten Blockierungsmittel für Hydroxylgruppen.

Für die Umsetzung wird das Blockierungsmittel mindestens stöchiometrisch in Bezug auf die Hydroxylgruppen eingesetzt, so dass die Hydroxylgruppen im Wesentlichen vollständig blockiert werden und der erhaltene Polyether somit im Wesentlichen frei ist von Hydroxylgruppen. Für die Blockierung werden für die jeweiligen Reaktivgruppen übliche Verfahren eingesetzt, gegebenenfalls unter Mitverwendung von Katalysatoren oder Lösemitteln. Falls bei der Blockierungsreaktion Abspalter entstehen, werden diese mit einer geeigneten Methode aus der Reaktionsmischung entfernt, insbesondere mittels Destillation.

Als Blockierungsmittel geeignet sind nucleophile Verbindungen, welche mit Hydroxylgruppen eine Additions- oder Substitutionsreaktion eingehen.

Geeignet sind insbesondere Vinylether, Carbonsäuren, Carbonsäurechloride, Carbonsäureester oder Carbonsäureanhydride, Diketen, 2,2,5-Trimethyl-4H-1,3-dioxin-4-on, Alkylacetoacetate, Dialkylcarbonate, (Meth)acrylamide, Methylenmalonate oder Cyanacrylate.

Bevorzugt sind Vinylether, wie insbesondere Methylvinylether, Ethylvinylether, Isopropylvinylether, Butylvinylether, Isobutylvinylether, Isopropenylmethylether, Isopropenylethylether, 2,3-Dihydrofuran oder 3,4-Dihydro-2H-pyran, besonders bevorzugt Isobutylvinylether, 2,3-Dihydrofuran oder 3,4-Dihydro-2H-pyran, wobei blockierte Hydroxylgruppen in Form von Acetalgruppen entstehen. Bevorzugt wird die Umsetzung in Anwesenheit einer Säure als Katalysator durchgeführt, insbesondere Salzsäure, Schwefelsäure, Phosphorsäure oder einer Sulfonsäure, gegebenenfalls in Form eines sauren lonentauscherharzes.

Bevorzugt sind weiterhin Carbonsäuren, Carbonsäurechloride, Carbonsäureester oder Carbonsäureanhydride, wobei blockierte Hydroxylgruppen in Form von Estergruppen entstehen. Davon bevorzugt sind Carbonsäureanhydride oder Carbonsäureester, insbesondere Essigsäureanhydrid.

Im Fall von Essigsäureanhydrid als Blockierungsmittel wird bei der Umsetzung Essigsäurefreigesetzt, wobei blockierte Hydroxylgruppen in Form von Acetatgruppen entstehen.

Im Fall von Isopropenylacetat als Blockierungsmittel wird bei der Umsetzung Aceton freigesetzt, wobei ebenfalls blockierte Hydroxylgruppen in Form von Acetatgruppen entstehen.

Bevorzugt sind weiterhin Diketen, 2,2,5-Trimethyl-4H-1,3-dioxin-4-on oder sterisch gehinderte Alkylacetoacetate wie insbesondere tert.Butylacetoacetat, wobei blockierte Hydroxylgruppen in Form von Acetoestergruppen entstehen.

Bevorzugt sind weiterhin Dialkylcarbonate, insbesondere Dimethylcarbonat, wobei blockierte Hydroxylgruppen in Form von Carbonatgruppen, insbesondere Methylcarbonatgruppen, entstehen.

Als hydroxyfunktioneller Polyether geeignet sind insbesondere solche mit einer mittleren OH-Funktionalität im Bereich von 1 bis 3 und einem mittleren Molekulargewicht Mₙ im Bereich von 500 bis 2'500 g/mol, mehr bevorzugt 600 bis 2'000 g/mol, insbesondere 700 bis 1'500 g/mol.

Bevorzugt sind Polyoxypropylen-Monole mit einer OH-Zahl im Bereich von 25 bis 90 mg KOH/g, bevorzugt 50 bis 80 mg KOH/g, insbesondere Alkohol-gestartete Polyoxypropylen-Monole, insbesondere gestartet von Methanol, Ethanol, Propanol, Isopropanol, n-Butanol, Isobutanol, tert.Butanol, Pentanol, Hexanol, 2-Ethylhexanol, Laurylalkohol, Myristylalkohol, Palmitylalkohol, Allylalkohol, Cyclohexanol, Benzylalkohol oder Phenol. Davon bevorzugt sind Alkylalkohol-gestartete Polyoxypropylenmonole, insbesondere gestartet von Methanol, Ethanol oder n-Butanol. Besonders bevorzugt sind n-Butanol-gestartete Polyoxypropylen-Monole mit einem mittleren Molekulargewicht Mₙ im Bereich von 650 bis 2'000 g/mol, insbesondere 700 bis 1'500 g/mol. n-Butanol-gestartete Polyoxypropylen-Monole sind kommerziell erhältlich, beispielsweise als Synalox^{®} 100-20B, Synalox^{®} 100-40B oder Synalox^{®} 100-85B (alle von DowDuPont Inc.).

Weiterhin bevorzugt sind Polyoxypropylen-Diole mit einer OH-Zahl im Bereich von 45 bis 155 mg KOH/g, bevorzugt 56 bis 125 mg KOH/g.

Weiterhin bevorzugt sind Trimethylolpropan- oder insbesondere Glycerin-gestartete, gegebenenfalls Ethylenoxid-terminierte, Polyoxypropylen-Triole mit einer mittleren OH-Funktionalität im Bereich von 2.2 bis 3 und einer OH-Zahl im Bereich von 90 bis 230 mg KOH/g.

Die bevorzugten Polyether mit blockierten Hydroxylgruppen sind aus gut verfügbaren Grundstoffen in einem einfachen Verfahren herstellbar, niedrigviskos und ermöglichen pastenförmige und gut handhabbare Thixotropiermittel, welche überraschenderweise schon in niedriger Menge zu einer starken Erhöhung der Fliessgrenze führen, ohne die Viskosität bzw. Auspressbarkeit des zu thixotropierenden Materials über Gebühr zu erhöhen .

Ein weiterer Gegenstand der Erfindung ist die Verwendung des erfindungsgemässen Thixotropiermittels in einer härtbaren Zusammensetzung zum Erhöhen der Fliessgrenze der Zusammensetzung, also zum sogenannten Thixotropieren der Zusammensetzung. Oft wird dieser Effekt auch als "Verdickung" bezeichnet, wobei damit aber nicht eine Erhöhung der Viskosität gemeint ist, sondern das Erhöhen der Fliessgrenze bzw. der Standfestigkeit. Eine solche thixotropierte Zusammensetzung kann auf einen geneigten Untergrund oder vertikal überhängend appliziert werden, ohne abzufliessen oder sich stark abzusenken.

Für diese Verwendung wird das Thixotropiermittel mit der härtbaren Zusammensetzung oder mit mindestens einem Bestandteil der härtbaren Zusammensetzung mit einer geeigneten Methode vermischt, bevorzugt unter Ausschluss von Feuchtigkeit, gegebenenfalls unter Vakuum.

Dabei bewirken insbesondere die im Thixotropiermittel enthaltenen Harnstoffverbindungen die erhöhte Fliessgrenze, während der Polyether mit blockierten Hydroxylgruppen einen verdünnenden und nach der Aushärtung weichmachenden Einfluss auf die Zusammensetzung ausübt.

Als härtbare Zusammensetzung bevorzugt ist eine Isocyanat- und/oder Silangruppen-haltige Zusammensetzung.

Besonders bevorzugt sind härtbare Zusammensetzungen, welche mindestens ein Isocyanat- und/oder Silangruppen-haltiges Polymer umfassen.

Die härtbare Zusammensetzung kann in Form einer einkomponentigen oder in Form einer mehrkomponentigen, insbesondere zweikomponentigen, Zusammensetzung vorliegen.

Als "einkomponentig" wird eine Zusammensetzung bezeichnet, bei welcher alle Bestandteile der Zusammensetzung im gleichen Gebinde vorliegen, welche als solche lagerstabil ist und welche mit Feuchtigkeit härtbar ist.

Als "zweikomponentig" wird eine Zusammensetzung bezeichnet, bei welcher die Bestandteile der Zusammensetzung in zwei verschiedenen Komponenten vorliegen, welche in voneinander getrennten Gebinden gelagert und erst kurz vor oder während der Applikation der Zusammensetzung miteinander vermischt werden.

Bevorzugt ist die härtbare Zusammensetzung einkomponentig und feuchtigkeitshärtend. Das erfindungsgemässe Thixotropiermittel ist besonders geeignet für einkomponentige Zusammensetzungen. Es ist unter Ausschluss von Feuchtigkeit zusammen mit diesen lagerstabil.

Besonders bevorzugt ist die Verwendung als Thixotropiermittel für härtbare Zusammensetzungen, welche eine besonders gute Standfestigkeit aufweisen und trotzdem ohne grossen Kraftaufwand aus einem Gebinde applizierbar sein sollen. Solche Eigenschaften sind insbesondere für pastöse Kleb- und Dichtstoffe gewünscht. Diese sollen gut aus einer Kartusche ausgepresst oder aus einem Hobbock oder Fass gepumpt werden können und dank hoher Standfestigkeit und kurzem Fadenzug eine unkomplizierte und saubere Applikation gewährleisten. Das beschriebene Thixotropiermittel ermöglicht phthalatfreie, pastöse einkomponentige Kleb- und Dichtstoffe, bei welchen ein horizontal von einer Wand abstehender Konus (Nase) mit etwa 2 bis 3 cm Grundfläche und etwa 5 bis 7 cm Länge so stehenbleibt, dass er sich nach der Applikation an der Spitze gemessen nur um wenige Millimeter absenkt.

Ein weiterer Gegenstand der Erfindung ist eine härtbare Zusammensetzung umfassend das erfindungsgemässe Thixotropiermittel und mindestens ein Isocyanat- und/oder Silangruppen-haltiges Polymer.

Das Isocyanat- und/oder Silangruppen-haltige Polymer weist bevorzugt ein mittleres Molekulargewicht Mₙ im Bereich von 1'000 bis 30'000 g/mol, insbesondere 2'000 bis 20'000 g/mol, auf.

Bevorzugt ist es bei Raumtemperatur flüssig.

In einer bevorzugten Ausführungsform enthält die Zusammensetzung mindestens ein Isocyanatgruppen-haltiges Polymer. Eine solche Zusammensetzung wird auch als Polyurethanzusammensetzung bezeichnet.

Ein geeignetes Isocyanatgruppen-haltiges Polymer wird insbesondere erhalten aus der Umsetzung von mindestens einem Polyol mit einer überstöchiometrischen Menge von mindestens einem Diisocyanat. Die Umsetzung wird bevorzugt unter Feuchtigkeitsausschluss bei einer Temperatur im Bereich von 20 bis 160°C, insbesondere 40 bis 140°C, durchgeführt, gegebenenfalls in Anwesenheit geeigneter Katalysatoren.

Das NCO/OH-Verhältnis liegt bevorzugt im Bereich von 1.3/1 bis 10/1. Das nach der Umsetzung der OH-Gruppen im Reaktionsgemisch verbleibende monomere Diisocyanat kann entfernt werden, insbesondere mittels Destillation.

Für den Fall, dass überschüssiges monomeres Diisocyanat mittels Destillation entfernt wird, liegt das NCO/OH-Verhältnis bei der Umsetzung bevorzugt im Bereich von 4/1 bis 7/1, und das erhaltene Isocyanatgruppen-haltige Polymer enthält nach der Destillation bevorzugt höchstens 0.5 Gewichts-%, besonders bevorzugt höchstens 0.3 Gewichts-%, monomeres Diisocyanat. Dabei wird monomeres Diisocyanat insbesondere mittels Kurzwegdestillation im Vakuum entfernt.

Für den Fall, dass kein überschüssiges monomeres Diisocyanat aus dem Polymer entfernt wird, liegt das NCO/OH-Verhältnis bei der Umsetzung bevorzugt im Bereich von 1.3/1 bis 2.5/1.

Das erhaltene Polymer hat bevorzugt einen Gehalt an Isocyanatgruppen im Bereich von 0.5 bis 10 Gewichts-%, insbesondere 1 bis 5 Gewichts-%, besonders bevorzugt 1 bis 3 Gewichts-%, und ein mittleres Molekulargewicht Mₙ im Bereich von 1'500 bis 20'000 g/mol, insbesondere 2'000 bis 15'000 g/mol. Gegebenenfalls wird das Polymer unter Mitverwendung von Weichmachern oder Lösemitteln hergestellt, wobei die verwendeten Weichmacher oder Lösemittel keine gegenüber Isocyanaten reaktive Gruppen enthalten.

Bevorzugt sind aliphatische, cycloaliphatische oder aromatische Diisocyanate, insbesondere 1,6-Hexamethylendiisocyanat (HDI), 1-Isocyanato-3,3,5-trimethyl-5-isocyanatomethylcyclohexan (Isophorondiisocyanat oder IPDI), Perhydro-2,4'- und/oder -4,4'-diphenylmethandiisocyanat (H₁₂MDI), 4,4'-Diphenylmethandiisocyanat, gegebenenfalls mit Anteilen von 2,4'- und/oder 2,2'-Diphenylmethandiisocyanat (MDI), oder 2,4-Toluylendiisocyanat oder Gemische davon mit 2,6-Toluylendiisocyanat (TDI).

Besonders bevorzugt ist HDI, IPDI, MDI oder TDI, oder Gemische davon.

Geeignete Polyole sind handelsübliche Polyole oder Mischungen davon, insbesondere
- Polyetherpolyole, insbesondere Polyoxyalkylendiole und/oder Polyoxyalkylentriole, insbesondere Polymerisationsprodukte von Ethylenoxid oder 1,2-Propylenoxid oder 1,2- oder 2,3-Butylenoxid oder Oxetan oder Tetrahydrofuran oder Mischungen davon, wobei diese mit Hilfe eines Startermoleküls mit zwei oder drei aktiven Wasserstoffatomen polymerisiert sein können, insbesondere einem Startermolekül wie Wasser, Ammoniak oder einer Verbindung mit mehreren OH- oder NH-Gruppen wie beispielsweise 1,2-Ethandiol, 1,2- oder 1,3-Propandiol, Neopentylglykol, Diethylenglykol, Triethylenglykol, die isomeren Dipropylenglykole oder Tripropylenglykole, die isomeren Butandiole, Pentandiole, Hexandiole, Heptandiole, Octandiole, Nonandiole, Decandiole, Undecandiole, 1,3- oder 1,4-Cyclohexandimethanol, Bisphenol A, hydriertes Bisphenol A, 1,1,1-Trimethylolethan, 1,1,1-Trimethylolpropan, Glycerin oder Anilin, oder Mischungen der vorgenannten Verbindungen. Ebenfalls geeignet sind Polyetherpolyole mit darin dispergierten Polymerpartikeln, insbesondere solche mit Styrol-Acrylnitril-Partikeln (SAN) oder Polyharnstoff- bzw. Polyhydrazodicarbonamid-Partikeln (PHD).
   Bevorzugte Polyetherpolyole sind Polyoxypropylendiole oder Polyoxypropylen-triole, oder sogenannte Ethylenoxid-terminierte (EO-capped bzw. EO-tipped) Polyoxypropylendiole oder -triole. Letztere sind Polyoxyethylen-polyoxypropylen-Mischpolyole, die insbesondere dadurch erhalten werden, dass Polyoxypropylendiole oder -triole nach Abschluss der Polypropoxylierungsreaktion mit Ethylenoxid weiter alkoxyliert werden und dadurch primäre Hydroxylgruppen aufweisen.
   Bevorzugte Polyetherpolyole weisen einen Ungesättigtheitsgrad von weniger als 0.02 mEq/g, insbesondere weniger als 0.01 mEq/g auf.
- Polyesterpolyole, auch Oligoesterole genannt, hergestellt nach bekannten Verfahren, insbesondere der Polykondensation von Hydroxycarbonsäuren bzw. Lactonen oder der Polykondensation von aliphatischen und/oder aromatischen Polycarbonsäuren mit zwei- oder mehrwertigen Alkoholen. Bevorzugt sind Polyesterdiole aus der Umsetzung von zweiwertigen Alkoholen wie insbesondere 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole, mit organischen Dicarbonsäuren oder deren Anhydride oder Ester, wie insbesondere Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure, 1,2-Cyclohexandicarbonsäure, 1,3-Cyclohexandicarbonsäure oder 1,4-Cyclohexandicarbonsäure oder Mischungen der vorgenannten Säuren, oder Polyesterpolyole aus Lactonen wie insbesondere ε-Caprolacton. Besonders bevorzugt sind Polyesterpolyole aus Adipinsäure oder Sebacinsäure oder Dodecandicarbonsäure und Hexandiol oder Neopentylglykol.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei OH-Gruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- oder Polymethacrylatpolyole.
- Polyhydroxyfunktionelle Fette oder Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie insbesondere polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen oder Isopren, insbesondere polyhydroxyfunktionelle Acrylonitril/ Butadien-Copolymere, wie sie insbesondere aus Epoxiden oder Aminoalkoholen und carboxylterminierten Acrylonitril/Butadien-Copolymeren (beispielsweise kommerziell erhältlich unter dem Namen Hypro^{®} CTBN oder CTBNX oder ETBN von Emerald Performance Materials) hergestellt werden können; oder hydrierte polyhydroxyfunktionelle Polymere oder Copolymere von Dienen.

Geeignet sind insbesondere auch Mischungen von Polyolen.

Bevorzugt sind Polyetherpolyole, Polyesterpolyole, Polycarbonatpolyole, Poly-(meth)acrylatpolyole oder Polybutadienpolyole.

Besonders bevorzugt sind Polyetherpolyole, Polyesterpolyole, insbesondere aliphatische Polyesterpolyole, oder Polycarbonatpolyole, insbesondere aliphatische Polycarbonatpolyole.

Insbesondere bevorzugt sind Polyetherpolyole, insbesondere Polyoxyalkylenpolyole.

Am meisten bevorzugt sind Polyoxypropylendi- oder -triole oder Ethylenoxid-terminierte Polyoxypropylendi- oder -triole.

Bevorzugt sind Polyole mit einem mittleren Molekulargewicht Mₙ im Bereich von 400 bis 20'000 g/mol, bevorzugt von 1'000 bis 15'000 g/mol.

Bevorzugt sind Polyole mit einer mittleren OH-Funktionalität im Bereich von 1.6 bis 3.

Bevorzugt sind bei Raumtemperatur flüssige Polyole.

Bei der Herstellung eines Isocyanatgruppen-haltigen Polymers können auch Anteile von zwei- oder mehrfunktionellen Alkoholen mitverwendet werden, insbesondere 1,2-Ethandiol, 1,2-Propandiol, 1,3-Propandiol, 2-Methyl-1,3-propandiol, 1,2-Butandiol, 1,3-Butandiol, 1,4-Butandiol, 1,3-Pentandiol, 1,5-Pentandiol, 3-Methyl-1,5-pentandiol, Neopentylglykol, Dibromneopentylglykol, 1,2-Hexandiol, 1,6-Hexandiol, 1,7-Heptandiol, 1,2-Octandiol, 1,8-Octandiol, 2-Ethyl-1,3-hexandiol, Diethylenglykol, Triethylenglykol, Dipropylenglykol, Tripropylenglykol, 1,3- oder 1,4-Cyclohexandimethanol, ethoxyliertes Bisphenol-A, propoxyliertes Bisphenol-A, Cyclohexandiol, hydriertes Bisphenol-A, Dimerfettsäurealkohole, 1,1,1-Trimethylolethan, 1,1,1-Trimethylolpropan, Glycerin, Pentaerythritol, Zuckeralkohole wie insbesondere Xylit, Sorbit oder Mannit oder Zucker wie insbesondere Saccharose oder alkoxylierte Derivate der genannten Alkohole oder Mischungen der genannten Alkohole.

Das Isocyanatgruppen-haltige Polymer weist bevorzugt ein mittleres Molekulargewicht Mₙ im Bereich von 1'500 bis 20'000 g/mol, insbesondere 2'000 bis 15'000 g/mol, auf.

Zusätzlich zu einem Isocyanatgruppen-haltigen Polymer kann die Zusammensetzung mindestens ein oligomeres Isocyanat oder eine bei Raumtemperatur flüssige Form von MDI enthalten.

Geeignete oligomere Isocyanate sind insbesondere HDI-Biurete wie Desmodur^{®} N 100 oder N 3200 (von Covestro AG), Tolonate^{®} HDB oder HDB-LV (von Vencorex Holding SAS) oder Duranate^{®} 24A-100 (von Asahi Kasei Corp.); HDI-Isocyanurate wie Desmodur^{®} N 3300, N 3600 oder N 3790 BA (alle von Covestro AG), Tolonate^{®} HDT, HDT-LV oder HDT-LV2 (von Vencorex Holding SAS), Duranate^{®} TPA-100 oder THA-100 (von Asahi Kasei Corp.) oder Coronate^{®} HX (von Tosoh Corp.); HDI-Uretdione wie Desmodur^{®} N 3400 (von Covestro AG); HDI-Iminooxadiazindione wie Desmodur^{®} XP 2410 (von Covestro AG); HDI-Allophanate wie Desmodur^{®} VP LS 2102 (von Covestro AG); IPDI-Isocyanurate wie beispielsweise in Lösung als Desmodur^{®} Z 4470 (von Covestro AG) oder in fester Form als Vestanat^{®} T1890/ 100 (von Evonik Industries AG); TDI-Oligomere wie Desmodur^{®} IL (von Covestro AG); oder gemischte Isocyanurate auf Basis TDI/HDI wie Desmodur^{®} HL (von Covestro AG).

Eine bei Raumtemperatur flüssige Form von MDI stellt entweder durch partielle chemische Modifizierung - insbesondere Carbodiimidisierung bzw. Uretoniminbildung oder Adduktbildung mit Polyolen - verflüssigtes 4,4'-MDI dar, oder es ist ein durch Abmischen gezielt herbeigeführtes oder durch den Herstellungsprozess bedingtes Gemisch von 4,4'-MDI mit anderen MDI-Isomeren (2,4'-MDI und/oder 2,2'-MDI), und/oder MDI-Oligomeren und/oder MDI-Homologen (polymeres MDI oder PMDI).

In einer bevorzugten Ausführungsform der Erfindung umfasst die härtbare Zusammensetzung zusätzlich zu mindestens einem Isocyanatgruppen-haltigen Polymer mindestens einen latenten Härter. Solche Polyurethanzusammensetzungen sind besonders wenig anfällig auf Blasenbildung während der Aushärtung. Bevorzugte latente Härter sind Ketimine, Aldimine oder Oxazolidine, insbesondere Oxazolidine oder Aldimine, am meisten bevorzugt Aldimine.

Bevorzugt ist ein Aldimin der Formel 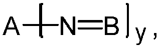 wobei y für 2 oder 3, A für einen organischen Rest mit 2 bis 23 C-Atomen und B für einen organischen Rest mit 6 bis 30 C-Atomen stehen.

Bevorzugt steht A für einen gegebenenfalls cyclische Anteile aufweisenden Alkylen-Rest oder einen zwei- oder dreiwertigen Polyoxyalkylen-Rest mit 5 bis 15 C-Atomen, insbesondere für 1,6-Hexylen, (1,5,5-Trimethylcyclohexan-1-yl)methan-1,3 oder α,ω-Polyoxypropylen mit einem mittleren Molekulargewicht Mₙ im Bereich von 170 bis 300 g/mol oder Trimethylolpropan-gestartetes Tris(ω-polyoxypropylen) mit einem mittleren Molekulargewicht Mₙ im Bereich von 330 bis 500 g/mol. Bevorzugt steht B für einen organischen Rest mit 7 bis 22 C-Atomen, insbesondere für 2,2-Dimethyl-3-acetoxypropyliden, 2,2-Dimethyl-3-lauroyloxypropyliden, 2,2-Dimethyl-3-(N-morpholino)propyliden, Benzyliden oder Alkyl-substituiertes Benzyliden, insbesondere 4-Decylbenzyliden, 4-Undecylbenzyliden, 4-Dodecylbenzyliden, 4-Tridecylbenzyliden oder 4-Tetradecylbenzyliden, in denen die 4-Alkyl-Reste mehrheitlich verzweigt vorliegen.

Besonders bevorzugt steht B für einen Rest mit mindestens 15 C-Atomen, insbesondere für 2,2-Dimethyl-3-lauroyloxypropyliden oder Alkyl-substituiertes Benzyliden. Ein solches Aldimin ist geruchsfrei.

Ein Aldimin der Formel 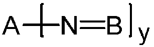 wird insbesondere erhalten durch Umsetzung eines Amins der Formel A-(NH₂)_{y} mit einem Aldehyd der Formel O=B unter Entfernung von Kondensationswasser.

Als Amin A-(NH₂)_{y} bevorzugt sind aliphatische oder cycloaliphatische primäre Di- oder Triamine, insbesondere 1,6-Hexamethylendiamin, Isophorondiamin, α,ω-Polyoxypropylendiamine mit einem mittleren Molekulargewicht Mₙ im Bereich von 200 bis 350 g/mol, insbesondere Jeffamine^{®} D-230 (von Huntsman Corp.), oder Trimethylolpropan-gestartetes Tris(ω-polyoxypropylenamin), insbesondere Jeffamine^{®} T-403 (von Huntsman Corp.).

Als Aldehyd O=B bevorzugt sind Aldolester von Carbonsäuren, insbesondere 2,2-Dimethyl-3-acetoxypropanal,-2,2-Dimethyl-3-lauroxyloxypropanal, 2,2-Dimethyl-3-(N-morpholino)propanal, Benzaldehyd oder mit Alkyl-Resten substituierte Benzaldehyde, insbesondere 4-Decylbenzaldehyd, 4-Undecylbenzaldehyd, 4-Dodecylbenzaldehyd, 4-Tridecylbenzaldehyd oder 4-Tetradecylbenzaldehyd, in denen die 4-Alkyl-Reste mehrheitlich verzweigt vorliegen, sowie Gemische dieser mit Alkyl-Resten substituierten Benzaldehyde.

Bei Kontakt mit Feuchtigkeit werden aus dem latenten Härter Aminogruppen und gegebenenfalls Hydroxylgruppen freigesetzt, welche mit Isocyanaten reagieren und als Vernetzer wirken. Dabei wird ein Aldehyd oder Keton freigesetzt.

Im Fall der bevorzugten Aldehyde der Formel O=B, bei welchen B für einen langkettigen Rest, insbesondere für einen Rest mit 15 oder mehr C-Atomen steht, verursacht dieser keine Geruchsprobleme und verbleibt nach der Aushärtung in der Zusammensetzung, wo er gut verträglich ist und ebenfalls als Weichmacher wirkt.

Gegenüber der direkten Reaktion von Wasser mit Isocyanaten weist die Vernetzung über latente Härter den Vorteil auf, dass dabei kein CO₂ abgespalten wird, was die Tendenz zur Bildung von Blasen bei der Aushärtung stark reduziert.

In einer weiteren bevorzugten Ausführungsform enthält die härtbare Zusammensetzung mindestens ein Silangruppen-haltiges organisches Polymer. Ein solches Polymer wird auch als "silanmodifiziertes Polymer" (SMP) und eine solche Zusammensetzung somit auch als SMP-Zusammensetzung bezeichnet.

Das Silangruppen-haltige organische Polymer weist bevorzugt Silangruppen der Formel 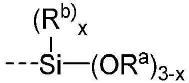 auf,
wobei
R^{a} für einen linearen oder verzweigten, einwertigen Kohlenwasserstoffrest mit 1 bis 5 C-Atomen, insbesondere für Methyl oder Ethyl, steht,
R^{b} für einen linearen oder verzweigten, einwertigen Kohlenwasserstoffrest mit 1 bis 8 C-Atomen, insbesondere für Methyl, steht, und
x für einen Wert von 0 oder 1 oder 2, bevorzugt für 0 oder 1, insbesondere für 0, steht.

Dabei weisen Methoxysilangruppen den Vorteil auf, dass sie besonders reaktiv sind. Ethoxysilangruppen weisen den Vorteil auf, dass sie toxikologisch vorteilhaft und besonders lagerstabil sind.

Besonders bevorzugt sind Trimethoxysilangruppen, Dimethoxymethylsilangruppen oder Triethoxysilangruppen.

Am meisten bevorzugt sind Trimethoxysilangruppen oder Triethoxysilangruppen.

Ein bevorzugtes Silangruppen-haltiges organisches Polymer ist ein Polyolefin oder ein Polyester oder ein Polyamid oder ein Poly(meth)acrylat oder ein Polyether oder eine Mischform dieser Polymere. Die Silangruppen können seitlich in der Kette oder endständig stehen und sind über ein C-Atom an das organische Polymer gebunden.

Besonders bevorzugt ist das Silangruppen-haltige organische Polymer ein Silangruppen-haltiger Polyether.

Als "Silangruppen-haltiger Polyether" werden organische Polymere enthaltend mindestens eine Silangruppe bezeichnet, deren Polymerkette hauptsächlich Polyether-Einheiten, insbesondere 1,2-Oxypropylen-Einheiten, aufweisen. Neben den Polyether-Einheiten können insbesondere auch Urethangruppen, Harnstoffgruppen, Thiourethangruppen, Estergruppen oder Amidgruppen enthalten sein.

Der Silangruppen-haltige Polyether enthält bevorzugt mindestens 50 Gewichts-%, insbesondere mindestens 70 Gewichts-%, besonders bevorzugt mindestens 80 Gewichts-%, 1,2-Oxypropylen-Einheiten.

Verfahren zur Herstellung von geeigneten Silangruppen-haltigen Polyethern sind dem Fachmann bekannt.

In einem bevorzugten Verfahren sind Silangruppen-haltige Polyether erhältlich aus der Umsetzung von Allylgruppen-haltigen Polyethern mit Hydrosilanen, gegebenenfalls unter Kettenverlängerung mit beispielsweise Diisocyanaten.

In einem weiteren bevorzugten Verfahren sind Silangruppen-haltige Polyether erhältlich aus der Copolymerisation von Alkylenoxiden und Epoxysilanen, gegebenenfalls unter Kettenverlängerung mit beispielsweise Diisocyanaten.

In einem weiteren bevorzugten Verfahren sind Silangruppen-haltige Polyether erhältlich aus der Umsetzung von Polyetherpolyolen mit Isocyanatosilanen, gegebenenfalls unter Kettenverlängerung mit Diisocyanaten.

In einem weiteren bevorzugten Verfahren sind Silangruppen-haltige Polyether erhältlich aus der Umsetzung von Isocyanatgruppen-haltigen Polyethern mit Aminosilanen, Hydroxysilanen oder Mercaptosilanen. Silangruppen-haltige Polyether aus diesem Verfahren sind besonders bevorzugt. Dieses Verfahren ermöglicht den Einsatz einer Vielzahl von kommerziell gut verfügbaren, kostengünstigen Ausgangsmaterialien, womit unterschiedliche Polymereigenschaften erhalten werden können, insbesondere eine hohe Dehnbarkeit, eine hohe Festigkeit, ein niedriges Elastizitätsmodul, ein tiefer Glasübergangspunkt oder eine hohe Witterungsbeständigkeit.

Besonders bevorzugt ist der Silangruppen-haltige Polyether erhältlich aus der Umsetzung von Isocyanatgruppen-haltigen Polyethern mit Aminosilanen und/oder Hydroxysilanen und/oder Mercaptosilanen.

Geeignete Isocyanatgruppen-haltige Polyether sind insbesondere erhältlich aus der Umsetzung von Polyetherpolyolen, insbesondere Polyoxyalkylendiolen oder Polyoxyalkylentriolen, bevorzugt Polyoxypropylendiolen oder Polyoxypropylen-triolen, mit einer überstöchiometrischen Menge an Diisocyanaten.

Bevorzugt wird die Umsetzung zwischen dem Diisocyanat und dem Polyetherpolyol unter Feuchtigkeitsausschluss bei einer Temperatur von 50°C bis 160°C durchgeführt, gegebenenfalls in Anwesenheit geeigneter Katalysatoren, wobei das Diisocyanat so dosiert ist, dass dessen Isocyanatgruppen im Verhältnis zu den Hydroxylgruppen des Polyols im stöchiometrischen Überschuss vorhanden sind. Insbesondere wird der Überschuss an Diisocyanat so gewählt, dass nach der Umsetzung aller Hydroxylgruppen ein Gehalt an freien Isocyanatgruppen von 0.1 bis 5 Gewichts-%, bevorzugt 0.2 bis 4 Gewichts-%, besonders bevorzugt 0.3 bis 3 Gewichts-%, bezogen auf das gesamte Polymer, verbleibt.

Bevorzugte Diisocyanate sind die bereits vorgängig erwähnten. Besonders bevorzugt ist IPDI oder TDI. Meist bevorzugt ist IPDI. Damit werden Silangruppen-haltige Polyether mit besonders guter Lichtechtheit erhalten.

Speziell geeignet als Polyetherpolyole sind Polyoxypropylendiole mit einem Ungesättigtheitsgrad tiefer als 0.02 mEq/g, insbesondere tiefer als 0.01 mEq/g, und einem mittleren Molekulargewicht Mₙ im Bereich von 400 bis 25'000 g/mol, insbesondere 1'000 bis 20'000 g/mol.

Neben Polyetherpolyolen können anteilig auch andere Polyole eingesetzt werden, insbesondere Polyacrylatpolyole, sowie niedrigmolekulare Diole oder Triole.

Geeignete Aminosilane für die Umsetzung mit einem Isocyanatgruppen-haltigen Polyether sind primäre und insbesondere sekundäre Aminosilane. Bevorzugt sind 3-Aminopropyltrimethoxysilan, 3-Aminopropyldimethoxymethylsilan, 4-Aminobutyltrimethoxysilan, 4-Amino-3-methylbutyltrimethoxysilan, 4-Amino-3,3-dime-thylbutyltrimethoxysilan, N-Butyl-3-aminopropyltrimethoxysilan, N-Phenyl-3-aminopropyltrimethoxysilan, Addukte aus primären Aminosilanen wie 3-Aminopropyltrimethoxysilan, 3-Aminopropyldimethoxymethylsilan oder N-(2-Aminoethyl)-3-aminopropyltrimethoxysilan und Michael-Akzeptoren wie Acrylnitril, (Meth)acrylsäureestern, (Meth)acrylsäureamiden, Maleinsäure- oder Fumarsäurediestern, Citraconsäurediestern oder Itaconsäurediestern, insbesondere N-(3-Trimethoxysilylpropyl)aminobernsteinsäuredimethyl- oder -diethylester. Ebenfalls geeignet sind Analoga der genannten Aminosilane mit Ethoxygruppen anstelle der Methoxygruppen am Silicium.

Geeignete Hydroxysilane für die Umsetzung mit einem Isocyanatgruppen-haltigen Polyether sind insbesondere erhältlich aus der Addition von Aminosilanen an Lactone oder an cyclische Carbonate oder an Lactide.

Bevorzugte Hydroxysilane, welche auf diese Weise erhalten werden, sind N-(3-Triethoxysilylpropyl)-2-hydroxypropanamid, N-(3-Trimethoxysilylpropyl)-2-hydro-xypropanamid, N-(3-Triethoxysilylpropyl)-4-hydroxypentanamid, N-(3-Triethoxy-silylpropyl)-4-hydroxyoctanamid, N-(3-Triethoxysilylpropyl)-5-hydroxydecanamid oder N-(3-Triethoxysilylpropyl)-2-hydroxypropylcarbamat.

Weiterhin geeignete Hydroxysilane sind erhältlich aus der Addition von Aminosilanen an Epoxide oder aus der Addition von Aminen an Epoxysilane. Bevorzugte Hydroxysilane, welche auf diese Weise erhalten werden, sind 2-Morpholino-4(5)-(2-trimethoxysilylethyl)cyclohexan-1-ol, 2-Morpholino-4(5)-(2-triethoxysilylethyl)cyclohexan-1-ol oder 1-Morpholino-3-(3-(triethoxysilyl)propoxy)propan-2-ol.

Geeignete Mercaptosilane für die Umsetzung mit einem Isocyanatgruppen-haltigen Polyether sind insbesondere 3-Mercaptopropyltrimethoxysilan oder 3-Mercaptopropyltriethoxysilan.

Als Silangruppen-haltige Polyether sind auch kommerziell erhältliche Produkte geeignet, insbesondere die Folgenden: MS Polymer^{™} (von Kaneka Corp.; insbesondere die Typen S203H, S303H, S227, S810, MA903 und S943); MS Polymer^{™} bzw. Silyl^{™} (von Kaneka Corp.; insbesondere die Typen SAT010, SAT030, SAT200, SAX350, SAX400, SAX725, MAX450, MAX951); Excestar^{®} (von Asahi Glass Co. Ltd.; insbesondere die Typen S2410, S2420, S3430, S3630); SPUR+* (von Momentive Performance Materials Inc.; insbesondere die Typen 1010LM, 1015LM, 1050MM); Vorasil^{™} (von DowDuPont Inc.; insbesondere die Typen 602 und 604); Desmoseal^{®} (von Covestro AG; insbesondere die Typen S XP 2458, S XP 2636, S XP 2749, S XP 2774 und S XP 2821), TEGOPAC^{®} (von Evonik Industries AG; insbesondere die Typen Seal 100, Bond 150, Bond 250), Polymer ST (von Hanse Chemie AG/Evonik Industries AG, insbesondere die Typen 47, 48, 61, 61LV, 77, 80, 81); Geniosil^{®} STP (von Wacker Chemie AG; insbesondere die Typen E10, E15, E30, E35).

Besonders bevorzugt wird der Silangruppen-haltige Polyether erhalten aus der Umsetzung von mindestens einem Isocyanatgruppen-haltigen Polyether mit mindestens einem Aminosilan und/oder Hydroxysilan und/oder Mercaptosilan.

Bevorzugt ist das Aminosilan und/oder Hydroxysilan und/oder Mercaptosilan dabei ausgewählt aus der Gruppe bestehend aus N-(3-Trimethoxysilylpropyl)-aminobernsteinsäuredimethylester, N-(3-Trimethoxysilylpropyl)aminobernsteinsäurediethylester, N-(3-Triethoxysilylpropyl)aminobernsteinsäuredimethylester, N-(3-Triethoxysilylpropyl)aminobernsteinsäurediethylester, N-(3-Trimethoxysilylpropyl)-2-hydroxypropanamid, N-(3-Triethoxysilylpropyl)-2-hydroxypropanamid, 3-Mercaptopropyltrimethoxysilan und 3-Mercaptopropyltriethoxysilan.

Die bevorzugten Ausführungsformen des Silangruppen-haltigen organischen Polymers ermöglichen Zusammensetzungen mit guter Lagerstabilität, schneller Aushärtung und besonders guten mechanischen Eigenschaften, insbesondere hoher Elastizität und Dehnbarkeit bei guter Festigkeit, und hoher Wärmebeständigkeit.

Bevorzugt enthält die härtbare Zusammensetzung zusätzlich einen oder mehrere weitere Bestandteile, insbesondere ausgewählt aus der Gruppe bestehend aus Weichmachern, Füllstoffen, Haftvermittlern, Trocknungsmitteln und Katalysatoren.

Geeignete Weichmacher sind insbesondere Carbonsäureester wie Phthalate, insbesondere Diisononylphthalat (DINP), Diisodecylphthalat (DIDP) oder Di(2-propylheptyl)phthalat (DPHP), hydrierte Phthalate bzw. 1,2-Cyclohexandicarbonsäureester, insbesondere hydriertes Diisononylphthalat bzw. Diisononyl-1,2-cyclohexandicarboxylat (DINCH), Terephthalate, insbesondere Bis(2-ethylhexyl)terephthalat (DOTP) oder Diisononylterephthalat (DINT), hydrierte Terephthalate bzw. 1,4-Cyclohexandicarbonsäureester, insbesondere hydriertes Bis(2-ethylhexyl)terephthalat bzw. Bis(2-ethylhexyl)-1,4-cyclohexandicarboxylat oder hydriertes Diisononylterephthalat bzw. Diisononyl-1,4-cyclohexandicarboxylat, Isophthalate, Trimellitate, Adipate, insbesondere Dioctyladipat, Azelate, Sebacate, Benzoate, Glykolether, Glykolester, Weichmacher mit Polyetherstruktur wie insbesondere die beschriebenen Polyether mit blockierten Hydroxylgruppen, organische Phosphor- oder Sulfonsäureester, Polybutene, Polyisobutene oder von natürlichen Fetten oder Ölen abgeleitete Weichmacher, insbesondere epoxidiertes Soja- oder Leinöl.

Bevorzugte Weichmacher sind die beschriebenen Polyether mit blockierten Hydroxylgruppen.

Geeignete Füllstoffe sind insbesondere gemahlene oder gefällte Calciumcarbonate, welche gegebenenfalls mit Fettsäuren, insbesondere Stearaten, beschichtet sind, Baryte (Schwerspate), Quarzmehle, Quarzsande, Dolomite, Wollastonite, calcinierte Kaoline, Schichtsilikate wie Glimmer oder Talk, Zeolithe, Aluminiumhydroxide, Magnesiumhydroxide, Kieselsäuren inklusive hochdisperse Kieselsäuren aus Pyrolyseprozessen, Zemente, Gipse, Flugaschen, industriell hergestellte Russe, Graphit, Metall-Pulver, beispielsweise von Aluminium, Kupfer, Eisen, Silber oder Stahl, PVC-Pulver oder Hohlkugeln.

Bevorzugt sind Calciumcarbonate, welche gegebenenfalls mit Fettsäuren, insbesondere Stearaten, beschichtet sind, calcinierte Kaoline oder industriell hergestellte Russe.

Geeignete Haftvermittler sind insbesondere Aminosilane wie insbesondere 3-Aminopropyltrimethoxysilan, 3-Aminopropyldimethoxymethylsilan, N-(2-Aminoethyl)-3-aminopropyl-trimethoxysilan, N-(2-Aminoethyl)-3-aminopropyl-dimethoxymethylsilan, N-(2-Aminoethyl)-N'-[3-(trimethoxysilyl)propyl]ethylendiamin oder deren Analoga mit Ethoxy- anstelle von Methoxygruppen, weiterhin N-Phenyl-, N-Cyclohexyl- oder N-Alkylaminosilane, Mercaptosilane, Epoxysilane, (Meth)acrylosilane, Anhydridosilane, Carbamatosilane, Alkylsilane oder Iminosilane, oligomere Formen dieser Silane, Addukte aus primären Aminosilanen mit Epoxysilanen oder (Meth)acrylosilanen oder Anhydridosilanen, aminofunktionelle Alkylsilsesquioxane, insbesondere aminofunktionelles Methylsilsesquioxan oder aminofunktionelles Propylsilsesquioxan, oder Titanate.

Für Isocyanatgruppen-haltige Zusammensetzung sind insbesondere Epoxysilane wie insbesondere 3-Glycidoxypropyltrimethoxysilan oder 3-Glycidoxypropyltriethoxysilan, (Meth)acrylosilane, Anhydridosilane, Carbamatosilane, Alkylsilane oder Iminosilane oder oligomere Formen dieser Silane als Haftvermittler geeignet.

Geeignete Trocknungsmittel für Zusammensetzungen mit Silangruppen-haltigen Polymeren sind insbesondere Tetraethoxysilan, Vinyltrimethoxysilan, Vinyltriethoxysilan oder Organoalkoxysilane, welche in α-Stellung zur Silangruppe eine funktionelle Gruppe aufweisen, insbesondere N-(Methyldimethoxysilylmethyl)-O-methylcarbamat, (Methacryloxymethyl)silane, Methoxymethylsilane, Orthoameisensäureester, Calciumoxid oder Molekularsiebpulver.

Geeignete Trocknungsmittel für Isocyanatgruppen-haltige Zusammensetzungen sind insbesondere Molekularsiebpulver, Calciumoxid, hochreaktive Isocyanate wie p-Tosylisocyanat, monomere Diisocyanate oder Orthoameisensäureester.

Geeignete Katalysatoren sind Katalysatoren für die Vernetzung von Silangruppen, insbesondere Metallkatalysatoren wie insbesondere Verbindungen von Zinn, Titan, Zirkonium, Aluminium oder Zink, und/oder stickstoffhaltige Verbindungen. Bevorzugt sind Diorganozinn(IV)-Verbindungen wie insbesondere Dibutylzinn-(IV)diacetat, Dibutylzinn(IV)dilaurat, Dibutylzinn(IV)dineodecanoat, Dibutylzinn-(IV)bis(acetylacetonat) oder Dioctylzinn(IV)dilaurat, weiterhin Titan(IV)- oder Zirkonium(IV)- oder Aluminium(III)- oder Zink(II)-Komplexe mit insbesondere Alkoxy-, Carboxylat-, 1,3-Diketonat-, 1,3-Ketoesterat- oder 1,3-Ketoamidat-Liganden, insbesondere Organotitanate, weiterhin Amine, Amidine wie insbesondere 1,8-Diazabicyclo[5.4.0]undec-7-en (DBU), 1,5-Diazabicyclo[4.3.0]non-5-en (DBN), 6-Dibutylamino-1,8-diazabicyclo[5.4.0]undec-7-en, 6-Dibutylamino-1,8-diazabicyclo[5.4.0]undec-7-en, N,N'-Di-n-hexylacetamidin (DHA), 2-Methyl-1,4,5,6-tetrahydropyrimidin, 1,2-Dimethyl-1,4,5,6-tetrahydropyrimidin, 2,5,5-Trimethyl-1,4,5,6-tetrahydropyrimidin, N-(3-Trimethoxysilylpropyl)-4,5-dihydroimidazol, N-(3-Triethoxysilylpropyl)-4,5-dihydroimidazol, 1-(3-Dimethylaminopropyl)-2-methyl-1,4,5,6-tetrahydropyrimidin, 1-(3-Aminopropyl)-2-methyl-1,4,5,6-tetrahydropyrimidin oder Umsetzungsprodukte davon, oder Guanidine wie insbesondere 1-Butylguanidin, 1,1-Dimethylguanidin, 1,3-Dimethylguanidin, 1,1,3,3-Tetramethylguanidin (TMG), 2-(3-(Trimethoxysilyl)propyl)-1,1,3,3-tetramethylguanidin, 2-(3-(Methyldimethoxysilyl)propyl)-1,1,3,3-tetramethylguanidin, 2-(3-(Triethoxysilyl)-propyl)-1,1,3,3-tetramethylguanidin, 1,5,7-Triazabicyclo[4.4.0]dec-5-en (TBD), 7-Methyl-1,5,7-triazabicyclo[4.4.0]dec-5-en, 7-Cyclohexyl-1,5,7-triazabicyclo[4.4.0]-dec-5-en, 1-Phenylguanidin, 1-(o-Tolyl)guanidin (OTG), 1,3-Diphenylguanidin, 1,3-Di(o-tolyl)guanidin, 2-Guanidinobenzimidazol oder Guanidine aus der Umsetzung von Monoaminen, Polyaminen oder Aminosilanen mit Carbodiimiden, insbesondere Dicyclohexylcarbodiimid oder Diisopropylcarbodiimid, sowie Biguanide oder Imidazole.

Bevorzugt sind Organotitanate, insbesondere Bis(ethylacetoacetato)diisobutoxytitan(IV) (kommerziell erhältlich beispielsweise als Tyzor^{®} IBAY von Dorf Ketal), Bis(ethylacetoacetato)diisopropoxytitan(IV) (kommerziell erhältlich beispielsweise als Tyzor^{®} DC von Dorf Ketal), Bis(acetylacetonato)diisopropoxytitan(IV), Bis(acetylacetonato)diisobutoxytitan(IV), Tris(oxyethyl)aminisopropoxytitan(IV), Bis[tris-(oxyethyl)amin]diisopropoxytitan(IV), Bis(2-ethylhexan-1,3-dioxy)titan(IV), Tris[2-((2-aminoethyl)amino)ethoxy]ethoxytitan(IV), Bis(neopentyl(diallyl)oxy)diethoxytitan(IV), Tetra(isopropoxy)titanat, Tetra(n-butoxy)titanat, Tetra(2-ethylhexyloxy)-titanat oder Polybutyltitanat, insbesondere Bis(ethylacetoacetato)diisobutoxytitan(IV) oder Bis(ethylacetoacetato)diisopropoxy-titan(IV).

Weiterhin bevorzugt sind Amidine oder Guanidine, insbesondere DBU, 1-(3-Dimethylaminopropyl)-2-methyl-1,4,5,6-tetrahydropyrimidin, 1-(3-Aminopropyl)-2-methyl-1,4,5,6-tetrahydropyrimidin oder Umsetzungsprodukte davon, oder Guanidine aus der Umsetzung von Monoaminen, Polyaminen oder Aminosilanen mit Dicyclohexylcarbodiimid oder Diisopropylcarbodiimid.

Weiterhin bevorzugt sind Kombinationen dieser Katalysatoren, insbesondere Kombinationen aus mindestens einem Organotitanat und mindestens einem Amidin oder Guanidin.

Geeignete Katalysatoren sind weiterhin Katalysatoren für die Beschleunigung der Reaktion von Isocyanatgruppen, insbesondere Organozinn(IV)-Verbindungen wie insbesondere Dibutylzinndiacetat, Dibutylzinndilaurat, Dibutylzinndichlorid, Dibutylzinndiacetylacetonat, Dimethylzinndilaurat, Dioctylzinndiacetat, Dioctylzinndilaurat oder Dioctylzinndiacetylacetonat, Komplexverbindungen von Bismut(III) oder Zirkonium(IV), insbesondere mit Liganden ausgewählt aus Alkoholaten, Carboxylaten, 1,3-Diketonaten, Oxinat, 1,3-Ketoesteraten und 1,3-Ketoamidaten, oder tertiäre Aminogruppen enthaltende Verbindungen wie insbesondere 2,2'-Dimorpholinodiethylether (DMDEE).

Geeignete Katalysatoren sind weiterhin Katalysatoren für die Hydrolyse von latenten Härtern, insbesondere Carbonsäuren wie 2-Ethylhexansäure, Laurinsäure, Stearinsäure, Neodecansäure, Benzoesäure, Salicylsäure oder 2-Nitrobenzoesäure, organische Carbonsäureanhydride, Silylester von Carbonsäuren, organische Sulfonsäuren, Sulfonsäureester, andere organische oder anorganische Säuren oder Mischungen der vorgenannten Säuren oder Säureester. Bevorzugt sind aromatische Carbonsäuren wie Benzoesäure, 2-Nitrobenzoesäure oder insbesondere Salicylsäure.

Die härtbare Zusammensetzung kann weitere Zusätze enthalten, insbesondere:
- anorganische oder organische Pigmente, insbesondere Titandioxid, Chromoxide oder Eisenoxide;
- Fasern, insbesondere Glasfasern, Kohlefasern, Metallfasern, Keramikfasern, Kunststofffasern wie Polyamidfasern oder Polyethylenfasern, oder Naturfasern wie Wolle, Cellulose, Hanf oder Sisal;
- Nanofüllstoffe wie Graphen oder Carbon Nanotubes;
- Farbstoffe;
- weitere Katalysatoren, welche die Reaktion der Isocyanatgruppen und/oder Silangruppen beschleunigen, insbesondere Salze, Seifen oder Komplexe von Zinn(II), Eisen, Aluminium, Molybdän, Dioxomolybdän oder Kalium, insbesondere Aluminiumlactat, Aluminiumoleat oder Kaliumacetat; tertiäre Aminogruppen enthaltende Verbindungen, insbesondere N-Ethyldiisopropylamin, N,N,N',N'-Tetramethylalkylendiamine, Pentamethylalkylentriamine und höhere Homologe davon, Bis-(N,N-diethylaminoethyl)adipat, Tris(3-dimethylaminopropyl)amin, 1,4-Diazabicyclo[2.2.2]octan (DABCO), N-Alkylmorpholine, N,N'-Dimethylpiperazin; stickstoffaromatische Verbindungen wie 4-Dimethylaminopyridin, N-Methylimidazol, N-Vinylimidazol oder 1,2-Dimethylimidazol; organische Ammoniumverbindungen wie Benzyltrimethylammoniumhydroxid oder alkoxylierte tertiäre Amine; sowie sogenannte "delayed action"-Katalysatoren, welche Modifizierungen bekannter Metall- oder Aminkatalysatoren darstellen;
- weitere Rheologie-Modifizierer, insbesondere Schichtsilikate wie Bentonite, Derivate von Ricinusöl, hydriertes Ricinusöl, Polyamide, Polyurethane, pyrogene Kieselsäuren, Celluloseether oder hydrophob modifizierte Polyoxyethylene;
- Lösemittel, insbesondere Aceton, Methylacetat, tert.Butylacetat, 1-Methoxy-2-propylacetat, Ethyl-3-ethoxypropionat, Diisopropylether, Diethylenglykoldiethylether, Ethylenglykoldiethylether, Ethylenglykolmonobutylether, Ethylenglykolmono-2-ethylhexylether, Acetale wie Propylal, Butylal, 2-Ethylhexylal, Dioxolan, Glycerolformal oder 2,5,7,10-Tetraoxaundecan (TOU), Toluol, Xylol, Heptan, Octan, Naphtha, White Spirit, Petrolether oder Benzin, insbesondere Solvesso^{™}-Typen (von ExxonMobil Chemical Co.), sowie Propylencarbonat, Dimethylcarbonat, Butyrolacton, N-Methylpyrrolidon, N-Ethylpyrrolidon, p-Chlorobenzotrifluorid oder Benzotrifluorid;
- natürliche Harze, Fette oder Öle wie Kolophonium, Schellack, Leinöl, Ricinusöl oder Sojaöl;
- nicht-reaktive Polymere, insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid oder Magnesiumhydroxid, sowie insbesondere organische Phosphorsäureester wie insbesondere Triethylphosphat, Trikresylphosphat, Triphenylphosphat, Diphenylkresylphosphat, Isodecyldiphenylphosphat, Tris-(1,3-dichlor-2-propyl)phosphat, Tris(2-chlorethyl)phosphat, Tris(2-ethylhexyl)-phosphat, Tris(chlorisopropyl)phosphat, Tris(chlorpropyl)phosphat, isopropyliertes Triphenylphosphat, Mono-, Bis- oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- Additive, insbesondere Netzmittel, Verlaufmittel, Entschäumer, Entlüfter, Stabilisatoren gegen Oxidation, Wärme, Licht oder UV-Strahlung oder Biozide;
oder weitere üblicherweise in härtbaren Zusammensetzungen eingesetzte Substanzen.

Es kann sinnvoll sein, gewisse Substanzen vor dem Einmischen in die Zusammensetzung chemisch oder physikalisch zu trocknen.

Die härtbare Zusammensetzung ist bevorzugt frei von Phthalaten.

Die härtbare Zusammensetzung enthält bevorzugt einen Gehalt an erfindungsgemässem Thixotropiermittel im Bereich von 1 bis 30 Gewichts-%, insbesondere 2.5 bis 25 Gewichts-%.

Der Gehalt an erfindungsgemässem Thixotropiermittel richtet sich nach dem gewünschten Grad an Thixotropierung, was vor allem abhängig ist von der Verwendung der Zusammensetzung.

Die härtbare Zusammensetzung wird insbesondere unter Ausschluss von Feuchtigkeit hergestellt und bei Umgebungstemperatur in feuchtigkeitsdichten Gebinden aufbewahrt. Ein geeignetes feuchtigkeitsdichtes Gebinde besteht insbesondere aus einem gegebenenfalls beschichteten Metall und/oder Kunststoff und ist insbesondere ein Fass, ein Container, ein Hobbock, ein Eimer, ein Kanister, eine Büchse, ein Beutel, ein Schlauchbeutel, eine Kartusche oder eine Tube.

Die härtbare Zusammensetzung ist bevorzugt einkomponentig und feuchtigkeitshärtend. Sie ist bei geeigneter Verpackung und Aufbewahrung lagerstabil, typischerweise während mehreren Monaten bis zu einem Jahr oder länger.

Bei der Applikation der Zusammensetzung beginnt der Prozess der Aushärtung. Als Ergebnis davon entsteht die ausgehärtete Zusammensetzung.

Im Fall einer einkomponentigen feuchtigkeitshärtenden Zusammensetzung wird diese als solche appliziert und beginnt darauf unter dem Einfluss von Feuchtigkeit bzw. Wasser auszuhärten. Zur Beschleunigung der Aushärtung kann der Zusammensetzung bei der Applikation eine Beschleuniger-Komponente, welche Wasser und/oder einen Katalysator enthält oder freisetzt, zugemischt werden, oder die Zusammensetzung kann nach ihrer Applikation mit einer solchen Beschleuniger-Komponente in Kontakt gebracht werden.

Im Fall einer zweikomponentigen Zusammensetzung wird diese nach dem Vermischen der beiden Komponenten appliziert und beginnt dabei durch interne Reaktion auszuhärten, wobei die Aushärtung gegebenenfalls durch die Einwirkung von externer Feuchtigkeit vervollständigt wird. Das Vermischen der beiden Komponenten kann kontinuierlich oder batchweise mit dynamischen Mischern oder Statikmischern erfolgen.

Bei der Aushärtung reagieren vorhandene Isocyanatgruppen unter dem Einfluss von Feuchtigkeit miteinander und/oder mit gegebenenfalls in der Zusammensetzung vorhandenen weiteren Reaktivgruppen, insbesondere Hydroxylgruppen oder Aminogruppen. Weiterhin reagieren vorhandene Isocyanatgruppen mit hydrolysierenden Reaktivgruppen von gegebenenfalls vorhandenen latenten Härter. Vorhandene Silangruppen reagieren bei der Aushärtung unter dem Einfluss von Feuchtigkeit miteinander. Sie können bei Kontakt mit Feuchtigkeit zu Silanolgruppen (Si-OH-Gruppen) hydrolysieren. Vorhandene Silangruppen können mit vorhandenen Silanolgruppen zu Siloxangruppen (Si-O-Si-Gruppen) kondensieren.

Die zur Aushärtung einer feuchtigkeitshärtenden Zusammensetzung benötigte Feuchtigkeit gelangt bevorzugt aus der Luft (Luftfeuchtigkeit) durch Diffusion in die Zusammensetzung. Dabei bildet sich an den mit Luft in Kontakt stehenden Oberflächen der Zusammensetzung eine feste Schicht ausgehärteter Zusammensetzung (Haut). Die Aushärtung setzt sich entlang der Diffusionsrichtung von aussen nach innen fort, wobei die Haut zunehmend dicker wird und schliesslich die ganze applizierte Zusammensetzung umfasst. Die Feuchtigkeit kann zusätzlich oder vollständig auch aus einem oder mehreren Substrat(en), auf welche(s) die Zusammensetzung appliziert wurde, in die Zusammensetzung gelangen und/oder aus einer Beschleuniger-Komponente stammen, welche der Zusammensetzung bei der Applikation zugemischt oder nach der Applikation mit dieser in Kontakt gebracht wird, beispielsweise durch Bestreichen oder Besprühen.

Die härtbare Zusammensetzung wird bevorzugt bei Umgebungstemperatur appliziert, insbesondere im Bereich von etwa -10 bis 50°C, bevorzugt im Bereich von - 5 bis 45°C, insbesondere 0 bis 40°C.

Die Aushärtung der Zusammensetzung erfolgt bevorzugt ebenfalls bei Umgebungstemperatur.

Die härtbare Zusammensetzung verfügt über strukturviskose Eigenschaften, insbesondere eine erhöhte Fliessgrenze, welche mit dem erfindungsgemässen Thixotropiermittel in einem breiten Bereich einstellbar ist.

Die härtbare Zusammensetzung kann so formuliert sein, dass sie eine pastöse Konsistenz mit hoher Fliessgrenze aufweist, insbesondere für die Verwendung als Klebstoff oder Dichtstoff. Eine solche Zusammensetzung kann aufgespachtelt oder mittels einer geeigneten Vorrichtung unter Druck appliziert werden, beispielsweise mittels einer Kartuschenpistole oder einer Fasspumpe oder einem Applikationsroboter, wobei die Zusammensetzung insbesondere in Form einer Raupe mit einer im Wesentlichen runden oder dreieckigen Querschnittsfläche ausgetragen wird. Dabei entsteht beim Wegziehen des Applikationsgerätes nur ein sehr kurzer Faden (Fadenzug) und somit wenig Gefahr von Verschmutzung, und die Zusammensetzung hat eine niedrige Viskosität, wodurch sie mit wenig Kraftaufwand gefördert werden kannund somit auch mittels handbetriebenen Vorrichtungen, wie insbesondere mechanischen Kartuschenpistolen, gut applizierbar ist.

Die härtbare Zusammensetzung kann weiterhin so formuliert sein, dass sie nur leicht thixotrop ist, insbesondere für die Verwendung als Dichtmasse oder Beschichtung. Eine solche Zusammensetzung kann zur Applikation ausgegossen oder gespachtelt werden, insbesondere mittels Zahnspachtel, Maurerkelle, Rakel oder Rolle. In einem Arbeitsgang wird typischerweise eine Schichtdicke im Bereich von 0.5 bis 5 mm, insbesondere 1 bis 3 mm, aufgetragen.

Das erfindungsgemässe Thixotropiermittel löst in der Zusammensetzung keine Reaktionen aus, die durch Viskositätserhöhung zu einer eingeschränkten Verwendbarkeit führen, und zeigt auch keine Neigung zu Separation oder Migration während der Lagerung im Gebinde. Dadurch weist die härtbare Zusammensetzung eine sehr gute Lagerstabilität auf. Nach der Aushärtung der Zusammensetzung verbleibt das Thixotropiermittel in der Zusammensetzung. Die Harnstoffverbindungen wirken in der ausgehärteten Zusammensetzungen wie ein Füllstoff und der Polyether mit blockierten Hydroxylgruppen übt einen weichmachenden, flexibilisierenden Effekt aus, neigt nicht zu Migration und verursacht weder Geruch noch Fogging.

Die härtbare Zusammensetzung eignet sich für eine Vielzahl von Verwendungen. Bevorzugt ist eine Verwendung als Klebstoff oder Dichtstoff oder Beschichtung, wobei der Klebstoff oder Dichtstoff oder die Beschichtung insbesondere elastisch ist.

Als Klebstoff und/oder Dichtstoff ist die Zusammensetzung insbesondere geeignet für Klebe- und Abdichtungsanwendungen, insbesondere in der Bau- und Fertigungsindustrie oder im Fahrzeugbau, insbesondere für die Parkettverklebung, Anbauteilverklebung, Hohlraumversiegelung, Montage, Modulverklebung, Karosserieverklebung, Scheibenverklebung oder Fugenabdichtung.

Elastische Verklebungen im Fahrzeugbau sind beispielsweise das Ankleben von Teilen wie Kunststoffabdeckungen, Zierleisten, Flansche, Stosstangen, Führerkabinen oder andere Anbauteile, an die lackierte Karosserie eines Fahrzeugs, oder das Einkleben von Scheiben in die Karosserie, wobei die Fahrzeuge insbesondere Automobile, Lastkraftwagen, Busse, Schienenfahrzeuge oder Schiffe darstellen.

Als Dichtstoff ist die Zusammensetzung insbesondere geeignet für das elastische Abdichten von Fugen, Nähten oder Hohlräumen aller Art, insbesondere von Fugen am Bau wie Dilatationsfugen oder Anschlussfugen zwischen Bauteilen. Insbesondere für das Abdichten von Dilatationsfugen an Bauwerken ist ein Dichtstoff mit weichelastischen Eigenschaften besonders geeignet.

Als Beschichtung ist die Zusammensetzung geeignet zum Schutz von Böden oder Mauern, insbesondere als Beschichtung von Balkonen, Terrassen, Plätzen, Brücken, Parkdecks, oder zur Abdichtung von Dächern, oder im Innern von Gebäuden zur Wasserabdichtung, beispielsweise unter Fliesen oder Keramikplatten in Nasszellen oder Küchen, oder als Abdichtung in Auffangwannen, Kanälen, Schächten, Silos, Tänken oder Abwasserbehandlungsanlagen, oder als Nahtabdichtung oder Schutzbeschichtung für beispielsweise Rohre.

Sie kann auch zu Reparaturzwecken als Abdichtung oder Beschichtung verwendet werden, beispielsweise von undichten Dachmembranen oder nicht mehr tauglichen Bodenbelägen oder insbesondere als Reparaturmasse für hochreaktive Spritzabdichtungen.

Geeignete Substrate, welche mit der Zusammensetzung verklebt oder abgedichtet oder beschichtet werden können, sind insbesondere
- Glas, Glaskeramik, Beton, Mörtel, Faserzement, insbesondere Faserzement-Platten, Backstein, Ziegel, Gips, insbesondere Gips-Platten, oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Kupfer, Eisen, Stahl, Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen wie Phenol-, Melamin- oder Epoxidharzen gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Farben oder Lacke, insbesondere Automobildecklacke.

Die Substrate können bei Bedarf vor dem Applizieren vorbehandelt werden, insbesondere durch physikalische und/oder chemische Reinigungsverfahren oder das Aufbringen eines Aktivators oder eines Primers.

Verklebt und/oder abgedichtet werden können zwei gleichartige oder zwei verschiedene Substrate.

Aus der Applikation und Aushärtung der Zusammensetzung wird ein Artikel erhalten.

Ein weiterer Gegenstand der Erfindung ist somit ein Artikel, welcher mit der Zusammensetzung verklebt oder abgedichtet oder beschichtet ist.

Dieser Artikel kann ein Bauwerk des Hoch- oder Tiefbaus oder ein Teil davon sein, insbesondere eine Brücke, ein Dach, ein Treppenhaus oder eine Fassade, oder er kann ein industrielles Gut oder ein Konsumgut sein, insbesondere ein Fenster, ein Rohr, ein Rotorblatt einer Windkraftanlage, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter, oder ein Anbauteil davon.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" ("NK") wird eine Temperatur von 23±1°C und eine relative Luftfeuchtigkeit von 50±5% bezeichnet.

Die verwendeten Chemikalien waren, sofern nicht anders bezeichnet, von Sigma-Aldrich Chemie GmbH.

### Herstellung von Polyethern mit blockierten Hydroxylgruppen:

Die **Viskosität** wurde mit einem thermostatisierten Kegel-Platten-Viskosimeter Rheotec RC30 (Kegeldurchmesser 25 mm, Kegelwinkel 1°, Kegelspitze-Platten-Abstand 0.05 mm, Scherrate 10 s⁻¹) gemessen.

**Infrarotspektren** (FT-IR) wurden als unverdünnte Filme auf einem mit horizontaler ATR-Messeinheit mit Diamant-Kristall ausgestatteten FT-IR Gerät Nicolet iS5 von Thermo Scientific gemessen. Die Absorptionsbanden sind in Wellenzahlen (cm⁻¹) angegeben.

**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Echte und Pseudo-Kopplungsmuster wurden nicht unterschieden.

### Polyether-1: n-Butanol-gestartetes acetyliertes PPG-Monol mit mittlerem Molekulargewicht ca. 800 g/mol

120.00 g n-Butanol-gestartetes Polyoxypropylen-Monol (Synalox^{®} 100-20B, mittleres Molekulargewicht ca. 750 g/mol; vonDowDuPont Inc.) und 18.74 g Essigsäureanhydrid wurden in einem Rundkolben mit Destillieraufsatz unter Stickstoffatmosphäre vorgelegt. Dann wurde die Reaktionsmischung bei 130 °C unter leichtem Stickstoffstrom gerührt, wobei als Destillat Essigsäure aufgefangen wurde. Anschliessend wurden bei 80°C und 10 mbar Vakuum die flüchtigen Bestandteile aus der Reaktionsmischung entfernt. Es wurde eine klare, farblose Flüssigkeit mit einer Viskosität von 75 mPa·s bei 20 °C erhalten.

FT-IR: 2970, 2931, 2867, 1738, 1454, 1372, 1345, 1296, 1241, 1098, 1014, 959, 925, 866, 827.

¹H-NMR (CDCl₃): 5.02 (hept., 1 H, CH₂(CH₃)C*H*-OAc), 3.75 - 3.34 (2 x m, ca. 39 H, OC*H*₂C*H*(CH₃)O), 3.33 - 3.28 (m, 2 H, CH₃CH₂CH₂C*H*₂O), 2.04 (s, 3 H, O(CO)C*H*₃), 1.55 (quint., 2 H, CH₃CH₂C*H*₂CH₂O), 1.36 (sext., 2 H, CH₃C*H*₂CH₂CH₂O), 1.22 (d, 3 H, CH₂(C*H*₃)CH-OAc), 1.17 - 1.10 (m, ca. 36 H, OCH₂CH(C*H*₃)O), 0.91 (t, 3 H, C*H*₃CH₂CH₂CH₂O).

### Polyether-2: n-Butanol-gestartetes Polypropylenglykol mit 1-(Isobutoxy)ethoxy-Endgruppe und mittlerem Molekulargewicht von ca. 1'200 g/mol

300.00 g n-Butanol-gestartetes Polyoxypropylen-Monol mit mittlerem Molekulargewicht 1'100 g/mol (Synalox^{®} 100-40B, vonDowDuPont Inc.) und 0.17 g Methansulfonsäure (wasserfrei) wurden in einem Rundkolben unter Stickstoffatmosphäre vorgelegt. Dann wurden 28.16 g Isobutylvinylether (stabilisiert mit 0.1% Kaliumhydroxid) unter Rühren langsam zugetropft, so dass die Temperatur der Reaktionsmischung nicht über 70°C stieg, und anschliessen bei 70°C unter Stickstoffatmosphäre so lange gerührt, bis mittels IR- und GC-Spektrometrie keine Hydroxylgruppen mehr nachweisbar waren. Anschliessend wurden 0.07 g Natriummethylat zugegeben und eingerührt, gefolgt von 0.06 g Essigsäure. Dann wurden zuerst bei 80°C und 5 mbar und dann bei 100°C und 2 mbar Vakuum die flüchtigen Bestandteilen aus der Reaktionsmischung entfernt. Es wurde eine klare, gelbliche Flüssigkeit mit einer Viskosität von 205 mPa·s bei 20°C erhalten.

FT-IR: 2969, 2931, 2868, 1455, 1372, 1344, 1296, 1257, 1099, 1012, 924, 906, 867, 831.

### Polyether-3: Diacetyliertes PPG-Diol mit mittlerem Molekulargewicht ca. 1'100 g/mol

80.00 g Polyoxypropylen-Diol (Voranol ^{®} P 1010, OH-Zahl 110 mg KOH/g, von DowDuPont Inc.) und 18.74 g Essigsäureanhydrid wurden wie für **Polyether-1** beschrieben umgesetzt. Es wurde eine klare, farblose Flüssigkeit mit einer Viskosität von 145 mPa·s bei 20°C erhalten.

### Herstellung von Thixotropiermitteln:

### Thixotropiermittel T-1 (erfindungsgemäss):

In einem Vakuummischer wurden 300 g **Polyether-1** und 48 g 4,4'-Methylendiphenyldiisocyanat (Desmodur^{®} 44 MC L, von Covestro AG) vorgelegt, leicht aufgewärmt und anschliessend unter starkem Rühren 27 g n-Butylamin langsam zugetropft. Die entstehende Paste wurde unter Vakuum und Kühlung eine Stunde weitergerührt. Es wurde eine weisse, feinteilige, homogene, streichfähige Paste erhalten.

### Thixotropiermittel T-2 (erfindungsgemäss):

Hergestellt wie für Thixotropiermittel **T-1** beschrieben, wobei aber 300 g **Polyether-2** anstelle von **Polyether-1** eingesetzt wurden. Es wurde eine gelbliche, feinteilige, homogene, streichfähige Paste erhalten.

### Thixotropiermittel T-3 (erfindungsgemäss):

Hergestellt wie für Thixotropiermittel **T-1** beschrieben, wobei aber 300 g **Polyether-3** anstelle von **Polyether-1** eingesetzt wurden. Es wurde eine weisse, feinteilige, homogene, streichfähige Paste erhalten.

### Thixotropiermittel T-4 (Vergleichsbeispiel):

Hergestellt wie für Thixotropiermittel **T-1** beschrieben, wobei aber 300 g Diisodecylphthalat (Palatinol^{®} 10-P, von BASF SE) anstelle von **Polyether-1** eingesetzt wurden. Es wurde eine weisse, feinteilige, homogene, streichfähige Paste erhalten.

### Thixotropiermittel T-5 (Vergleichsbeispiel):

Hergestellt wie für Thixotropiermittel **T-1** beschrieben, wobei aber 300 g Diisononyl-1,2-cyclohexandicarboxylat (Hexamoll^{®} DINCH, von BASF SE) anstelle von **Polyether-1** eingesetzt wurden. Es wurde eine weisse, feinteilige, homogene, streichfähige Paste erhalten.

### Thixotropiermittel T-6 (Vergleichsbeispiel):

Hergestellt wie für Thixotropiermittel **T-1** beschrieben, wobei aber 300 g Di(2-ethylhexyl)adipat (Plastomoll^{®} DOA, von BASF SE) anstelle von **Polyether-1** eingesetzt wurden. Es wurde eine weisse, feinteilige, homogene, streichfähige Paste erhalten.

Einige Thixotropiermittel wurden wie folgt auf Standfestigkeit und Auspresskraft geprüft:
Die **Standfestigkeit** wurde bestimmt, indem im Normklima ca. 8 ml der Paste durch eine Kartuschenspitze mit Innendurchmesser 10 mm so auf eine vertikale Fläche aus Pappkarton aufgetragen wurde, dass eine etwa 50 mm horizontal vorstehende Nase mit einem Durchmesser von etwa 20 mm entstand. Nach 3 Stunden wurde bestimmt, wie weit sich die Nase an der Spitze gemessen aus der horizontalen Lage abgesenkt hatte. Ein Absinken von weniger als 2 mm wurde als "gut", 2 bis 5 mm als "mittel" und mehr als 5 mm als "schlecht" beurteilt.

Die **Auspresskraft** (5mm; 3mm, 2mm) wurde bestimmt, indem die Paste in eine handelsübliche Aluminiumkartusche gefüllt, darauf eine Düse mit Innendurchmesser 5 mm bzw. 3 mm bzw. 2 mm aufgeschraubt und mit einem Auspressgerät (Zwick/Roell Z005) die aufgewendete Kraft gemessen wurde, um das Thixotropiermittel mit einer Auspressgeschwindigkeit von 60 mm/min durch die jeweilige Düse auszupressen. Der angegebene Wert ist ein Mittelwert aus den Kräften, die nach einem Auspressweg von 22 mm, 24 mm, 26 mm und 28 mm gemessen wurden.

Die Resultate sind in der Tabelle 1 dargestellt.

**Tabelle 1: Eigenschaften der Thixotropiermittel T-1 und T-4.**

| **Thixotropiermittel** | | **T-1** (erfindungsgemäss) | **T-4** (Vergleichsbeispiel) |
|---|---|---|---|
| Standfestigkeit | | gut | gut |
| Auspresskraft [N] | 5mm | 518 | 566 |
| | 3mm | 696 | 760 |
| | 2mm | 854 | 951 |

### Herstellung von härtbaren Zusammensetzungen (einkomponentig):

### Polymer P1:

590 g Polyoxypropylen-Diol (Acclaim^{®} 4200, von Covestro AG; OH-Zahl 28.5 mg KOH/g), 1180 g Polyoxypropylenpolyoxyethylen-Triol (Caradol^{®} MD34-02, von Shell Chemicals Co.; OH-Zahl 35.0 mg KOH/g) und 230 g Isophorondiisocyanat (Vestanat^{®} IPDI, von Evonik Industries AG) wurden nach bekanntem Verfahren bei 80 °C zu einem bei Raumtemperatur flüssigen, Isocyanatgruppen-haltigen Polymer mit einem Gehalt an freien Isocyanatgruppen von 2.1 Gewichts-% umgesetzt.

### Polymer P2:

400 g Polyoxypropylen-Diol (Acclaim^{®} 4200, von Covestro AG; OH-Zahl 28.5 mg KOH/g) und 52 g 4,4'-Diphenylmethandiisocyanat (Desmodur^{®} 44 MC L, von Covestro AG) wurden nach bekanntem Verfahren bei 80°C zu einem bei Raumtemperatur flüssigen, NCO-terminierten Polymer mit einem Gehalt an freien Isocyanatgruppen von 1.85 Gewichts-% umgesetzt.

### Polymer P3:

3080 g Polyoxypropylen-Diol (Acclaim^{®} 4200, von Covestro AG; OH-Zahl 28.5 mg KOH/g), 1540 g Polyoxypropylenpolyoxyethylen-Triol (Caradol^{®} MD34-02, von Shell Chemicals Co.; OH-Zahl 35.0 mg KOH/g) und 385 g Toluylendiisocyanat (Desmodur^{®} T 80 P, Covestro AG) wurden bei 80°C nach bekanntem Verfahren zu einem bei Raumtemperatur flüssigen, NCO-terminierten Polyurethanpolymer mit einem Gehalt an freien Isocyanatgruppen von 1.5 Gew.-% umgesetzt.

### Aldimin-1: N,N'-Bis(2,2-dimethyl-3-lauroyloxypropyliden)-3-aminomethyl-3,5,5-trimethylcyclohexylamin

598 g (2.1 mol) 2,2-Dimethyl-3-lauroyloxypropanal wurden unter Stickstoffatmosphäre in einem Rundkolben vorgelegt. Dann wurden 170.3 g (1 mol) 3-Aminomethyl-3,5,5-trimethylcyclohexylamin (Vestamin^{®} IPD von Evonik Industries AG) unter gutem Rühren zugegeben und anschliessend die flüchtigen Bestandteile bei 80 °C und 10 mbar Vakuum entfernt. Erhalten wurden 732 g einer farblosen Flüssigkeit mit einem Amingehalt von 2.73 mmol N/g, was einem berechneten Aldimin-Equivalentgewicht von 367 g/mol entspricht.

### Zusammensetzungen Z1 und Z2

Für jede Zusammensetzung wurden die in Tabelle 2 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt.

Von jeder Zusammensetzung wurde die **Standfestigkeit** bestimmt, indem im Normklima ca. 8 ml der Zusammensetzung durch eine Kartuschenspitze mit Innendurchmesser 10 mm so auf eine vertikale Fläche aus Pappkarton aufgetragen wurde, dass eine etwa 50 mm horizontal vorstehende Nase mit einem Durchmesser von etwa 20 mm entstand. Nach der Aushärtung im Normklima wurde bestimmt, wie weit sich die Nase an der Spitze, gemessen aus der horizontalen Lage, abgesenkt hatte. Ein Absinken von weniger als 15 mm wurde als "gut", 15 bis 30 mm als "mittel" und mehr als 30 mm als "schlecht" beurteilt.

Die Resultate sind in Tabelle 2 angegeben.

Mit "(Ref.)" bezeichnete Zusammensetzungen sind Vergleichsbeispiele.

**Tabelle 2: Zusammensetzung (in Gewichtsteilen) und Eigenschaften von Z1 und Z2.**

| **Zusammensetzung** | **Z1** | **Z2 (Ref.)** |
|---|---|---|
| Polymer **P1** | 200.0 | 200.0 |
| Thixotropiermittel | **T-1** | **T-4** |
| | 200.0 | 200.0 |
| Standfestigkeit | gut | gut |

### Zusammensetzungen Z3 bis Z6

Für jede Zusammensetzung wurden die in Tabelle 3 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt.

Von jeder Zusammensetzung wurde die Fleckenbildung auf Karton als Mass für die Weichmachermigration bestimmt. Dazu wurde jede Zusammensetzung so auf ein Stück Pappkarton appliziert, dass sie eine runde Grundfläche von 15 mm Durchmesser und eine Höhe von 4 mm aufwies, und während 7 Tagen im NK gelagert. Um jede Zusammensetzung herum war danach auf dem Karton ein dunkler ovaler Fleck entstanden. Dessen Ausmasse (Höhe und Breite) wurden ausgemessen und in Tabellen 3 als **Migration** angegeben.

Mit "(Ref.)" bezeichnete Zusammensetzungen sind Vergleichsbeispiele.

**Tabelle 3: Zusammensetzung (in Gewichtsteilen) und Eigenschaften von Z3 bis Z6.**

| **Zusammensetzung** | | **Z3** | **Z4 (Ref.)** | **Z5 (Ref.)** | **Z6 (Ref.)** |
|---|---|---|---|---|---|
| Polymer **P2** | | 80.00 | 80.00 | 80.00 | 80.00 |
| Kreide¹ | | 80.00 | 80.00 | 80.00 | 80.00 |
| Thixotropiermittel | | **T-1** | **T-4** | **T-5** | **T-6** |
| | | 115.9 | 115.9 | 115.9 | 115.9 |
| 2,2'-Dimorpholinodiethylether | | 0.26 | 0.26 | 0.26 | 0.26 |
| **Migration** | Höhe | 0.5 | 0.5 | 1 | 3 |
| (7d) [mm] | Breite | 1 | 1 | 2 | 4 |

| | | | | | |
|---|---|---|---|---|---|
| ¹ Omyacarb^{®} 5 GU (von Omya AG) | | | | | |

### Zusammensetzungen Z7 bis Z9

Für jede Zusammensetzung wurden die in Tabelle 4 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt und unter Feuchtigkeitsausschluss aufbewahrt.

Jede Zusammensetzung wurde folgendermassen geprüft:
Die **Standfestigkeit** wurde bestimmt wie für Zusammensetzung **Z1** beschrieben. Zusätzlich wurde beurteilt, ob beim Wegziehen des Applikationswerkzeugs nach der Applikation der "Nase" ein langer Faden weggezogen wurde. Wenn kein Faden, sondern nur eine kurze Spitze von weniger als 10 mm entstand, wurde der **Fadenzug** mit "nein" beurteilt.

Als Mass für die Offenzeit wurde die Hautbildungszeit **(HBZ)** bestimmt. Dazu wurden einige Gramm der Zusammensetzung in einer Schichtdicke von ca. 2 mm auf Pappkarton aufgetragen und im Normklima die Zeitdauer bestimmt, bis beim leichten Antippen der Oberfläche der Zusammensetzung mittels einer Pipette aus LDPE erstmals keine Rückstände auf der Pipette mehr zurückblieben.

Die **Shore A** Härte wurde bestimmt nach DIN 53505 an während 14 Tagen im Normklima ausgehärteten Prüfkörpern.

Zur Bestimmung der mechanischen Eigenschaften wurde die Zusammensetzung auf eine PTFE-beschichteten Folie zu einem Film von 2 mm Dicke aufgebracht, dieser während 14 Tagen im Normklima gelagert, einige Hanteln mit einer Länge von 75 mm bei einer Steglänge von 30 mm und einer Stegbreite von 4 mm aus dem Film ausgestanzt und diese gemäss DIN EN 53504 bei einer Zuggeschwindigkeit von 200 mm/min auf **Zugfestigkeit** (Bruchkraft), **Bruchdehnung, E-Modul 5%** (bei 0.5-5% Dehnung) und **E-Modul 25%** (bei 0.5-25% Dehnung) geprüft. Der **Aspekt** wurde visuell an den hergestellten Filmen beurteilt. Als "schön" wurde ein nichtklebriger Film ohne Blasen bezeichnet.

Der **Geruch** wurde durch Riechen mit der Nase im Abstand von 2 cm an den frisch hergestellten Filmen beurteilt. "Nein" bedeutet, dass kein Geruch wahrnehmbar war.

Die Resultate sind in der Tabelle 4 angegeben.

**Tabelle 4: Zusammensetzung (in Gewichtsteilen) und Eigenschaften von Z7 bis Z9. ¹ Omyacarb^{®} 5-GU (von Omya AG) ² 5 Gewicht-% in Di(2-ethylhexyl)adipat**

| **Zusammensetzung** | **Z7** | **Z8** | **Z9** |
|---|---|---|---|
| Polymer **P3** | 28.00 | 28.00 | 28.00 |
| Aldimin-1 | 2.58 | 2.58 | 2.58 |
| **Polyether-1** | 14.00 | - | - |
| **Polyether-2** | - | 14.00 | - |
| **Polyether-3** | - | - | 14.00 |
| Thixotropiermittel | **T-1** | **T-2** | **T-3** |
| | 20.00 | 20.00 | 20.00 |
| Kreide ¹ | 33.89 | 33.89 | 33.89 |
| Salicylsäure-Lösung ² | 1.50 | 1.50 | 1.50 |
| Dibutylzinndilaurat | 0.03 | 0.03 | 0.03 |
| Standfestigkeit | gut | gut | gut |
| Fadenzug | nein | nein | nein |
| HBZ [min] | 25 | 25 | 25 |
| Shore A | 10 | 9 | 16 |
| Zugfestigkeit [MPa] | 1.14 | 1.22 | 1.37 |
| Bruchdehnung [%] | 809 | 776 | 784 |
| E-Modul 5% [MPa] | 0.52 | 0.44 | 0.54 |
| E-Modul 25% [MPa] | 0.26 | 0.26 | 0.45 |
| Aspekt | schön | schön | schön |
| Geruch | nein | nein | nein |

## Patentansprüche

1. Thixotropiermittel zur Erhöhung der Fliessgrenze einer härtbaren Zusammensetzung, wobei das Thixotropiermittel (i) mindestens eine Harnstoffverbindung aus der Umsetzung von mindestens einem Isocyanat mit mindestens einem Amin und (ii) mindestens einen Polyether mit blockierten Hydroxylgruppen umfasst, welcher frei von Urethangruppen ist.

2. Thixotropiermittel gemäss Anspruch 1, **dadurch gekennzeichnet, dass** die Harnstoffverbindung die Formel (I) aufweist, 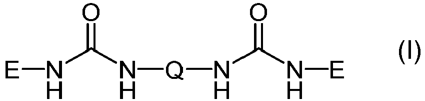 wobei
E für einen Alkyl- oder Cycloalkyl-Rest mit 1 bis 12 C-Atomen steht, und Q für einen zweiwertigen Kohlenwasserstoff-Rest mit 6 bis 15 C-Atomen steht.

3. Thixotropiermittel gemäss Anspruch 1 oder 2, **dadurch gekennzeichnet, dass** das Thixotropiermittel in Form einer bei Raumtemperatur standfesten, streichfähigen Paste vorliegt.

4. Thixotropiermittel gemäss einem der Ansprüche 1 bis 3, **dadurch gekennzeichnet, dass** 5 bis 25 Gewichts-% Harnstoffverbindungen und 50 bis 95 Gewichts-% Polyether mit blockierten Hydroxylgruppen enthalten sind.

5. Thixotropiermittel gemäss einem der Ansprüche 1 bis 4, **dadurch gekennzeichnet, dass** der Polyether mit blockierten Hydroxylgruppen eine Viskosität bei 20°C im Bereich von 30 bis 500 mPa·s aufweist, wobei die Viskosität bestimmt wird mit einem Kegel-Platten-Viskosimeter mit Kegeldurchmesser 25 mm, Kegelwinkel 1°, Kegelspitze-Platten-Abstand 0.05 mm bei einer Scherrate von 10 s⁻¹.

6. Thixotropiermittel gemäss einem der Ansprüche 1 bis 5, **dadurch gekennzeichnet, dass** der Polyether im Mittel 1 bis 3 blockierte Hydroxylgruppen pro Molekül aufweist.

7. Thixotropiermittel gemäss einem der Ansprüche 1 bis 6, **dadurch gekennzeichnet, dass** die blockierten Hydroxylgruppen ausgewählt sind aus der Gruppe bestehend aus Acetal-, Ester-, Acetoester- und Carbonatgruppen.

8. Thixotropiermittel gemäss einem der Ansprüche 1 bis 7, **dadurch gekennzeichnet, dass** im Polyether 70 bis 100 Gewichts-% der Repetiereinheiten aus 1,2-Propylenoxy-Gruppen und 0 bis 30 Gewichts-% der Repetiereinheiten aus 1,2-Ethylenoxy-Gruppen bestehen.

9. Thixotropiermittel gemäss einem der Ansprüche 1 bis 8, **dadurch gekennzeichnet, dass** der Polyether mit blockierten Hydroxylgruppen ein mittleres Molekulargewicht Mₙ im Bereich von 600 bis 2'500 g/mol aufweist, bestimmt mittels Gelpermeationschromatographie (GPC) gegen Polystyrol als Standard mit Tetrahydrofuran als mobiler Phase, Brechungsindex-Detektor und Auswertung ab 200 g/mol.

10. Thixotropiermittel gemäss einem der Ansprüche 1 bis 9, **dadurch gekennzeichnet, dass** der Polyether mit blockierten Hydroxylgruppen abgeleitet ist von mindestens einem hydroxyfunktionellen Polyether ausgewählt aus der Gruppe bestehend aus
- Alkohol-, insbesondere n-Butanol-, gestarteten Polyoxypropylen-Monolen mit einer OH-Zahl im Bereich von 25 bis 90 mg KOH/g,
- Polyoxypropylen-Diolen mit einer OH-Zahl im Bereich von 45 bis 155 mg KOH/g und
- Trimethylolpropan- oder insbesondere Glycerin-gestarteten, gegebenenfalls Ethylenoxid-terminierten, Polyoxypropylen-Triolen mit einer mittleren OH-Funktionalität im Bereich von 2.2 bis 3 und einer OH-Zahl im Bereich von 90 bis 230 mg KOH/g.

11. Herstellung des Thixotropiermittels gemäss einem der Ansprüche 1 bis 10, **dadurch gekennzeichnet, dass** die Umsetzung zwischen dem Isocyanat und dem Amin im Polyether mit blockierten Hydroxylgruppen durchgeführt wird.

12. Verwendung des Thixotropiermittels gemäss einem der Ansprüche 1 bis 10 in einer härtbaren Zusammensetzung zum Erhöhen der Fliessgrenze der Zusammensetzung.

13. Härtbare Zusammensetzung umfassend das Thixotropiermittels gemäss einem der Ansprüche 1 bis 10 und mindestens ein Isocyanat- und/oder Silangruppen-haltiges Polymer.

14. Härtbare Zusammensetzung gemäss Anspruch 13, **dadurch gekennzeichnet, dass** sie einkomponentig und feuchtigkeitshärtend ist.

15. Artikel, welcher mit der härtbaren Zusammensetzung gemäss einem der Ansprüche 13 bis 14 verklebt oder abgedichtet oder beschichtet ist.

## Claims

1. Thixotropic agent for increasing the yield point of a curable composition, wherein the thixotropic agent comprises (i) at least one urea compound from the reaction of at least one isocyanate with at least one amine and (ii) at least one polyether having blocked hydroxyl groups which is free of urethane groups.

2. Thixotropic agent according to Claim 1, **characterized in that** the urea compound has the formula (I) 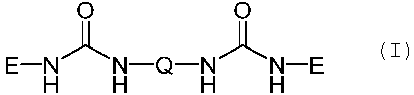 where
E is an alkyl or cycloalkyl radical having 1 to 12 carbon atoms, and
Q is a divalent hydrocarbyl radical having 6 to 15 carbon atoms.

3. Thixotropic agent according to Claim 1 or 2, **characterized in that** the thixotropic agent takes the form of a spreadable paste which is firm at room temperature.

4. Thixotropic agent according to any of Claims 1 to 3, **characterized in that** 5% to 25% by weight of urea compounds and 50% to 95% by weight of polyethers having blocked hydroxyl groups are present.

5. Thixotropic agent according to any of Claims 1 to 4, **characterized in that** the polyether having blocked hydroxyl groups has a viscosity at 20°C in the range from 30 to 500 mPa·s, where the viscosity is determined with a cone-plate viscometer having cone diameter of 25 mm, cone angle of 1°, cone tip-plate distance of 0.05 mm, at a shear rate of 10 s⁻¹.

6. Thixotropic agent according to any of Claims 1 to 5, **characterized in that** the polyether has an average of 1 to 3 blocked hydroxyl groups per molecule.

7. Thixotropic agent according to any of Claims 1 to 6, **characterized in that** the blocked hydroxyl groups are selected from the group consisting of acetal, ester, aceto ester and carbonate groups.

8. Thixotropic agent according to any of Claims 1 to 7, **characterized in that** 70% to 100% by weight of the repeat units in the polyether consist of 1,2-propyleneoxy groups, and 0% to 30% by weight of the repeat units in the polyether consist of 1,2-ethyleneoxy groups.

9. Thixotropic agent according to any of Claims 1 to 8, **characterized in that** the polyether having blocked hydroxyl groups has an average molecular weight Mₙ in the range from 600 to 2'500 g/mol, determined by means of gel permeation chromatography (GPC) versus polystyrene as standard with tetrahydrofuran as mobile phase, refractive index detector and evaluation from 200 g/mol.

10. Thixotropic agent according to any of Claims 1 to 9, **characterized in that** the polyether having blocked hydroxyl groups is derived from at least one hydroxyfunctional polyether selected from the group consisting of
- alcohol-started, especially n-butanol-started, polyoxypropylene monools having an OH number in the range from 25 to 90 mg KOH/g,
- polyoxypropylene diols having an OH number in the range from 45 to 155 mg KOH/g and
- trimethylolpropane-started or especially glycerol-started, optionally ethylene oxide-terminated, polyoxypropylene triols having an average OH functionality in the range from 2.2 to 3 and an OH number in the range from 90 to 230 mg KOH/g.

11. Preparation of the thixotropic agent according to any of Claims 1 to 10, **characterized in that** the reaction between the isocyanate and the amine is performed in the polyether having blocked hydroxyl groups.

12. Use of the thixotropic agent according to any of Claims 1 to 10 in a curable composition for increasing the yield point of the composition.

13. Curable composition comprising the thixotropic agent according to any of Claims 1 to 10 and at least one polymer containing isocyanate and/or silane groups.

14. Curable composition according to Claim 13, **characterized in that** it is a one-component moisturecuring composition.

15. Article bonded or sealed or coated with the curable composition according to either of Claims 13 and 14.

## Revendications

1. Agent thixotropique destiné à augmenter la limite d'écoulement d'une composition durcissable, l'agent thixotropique comprenant (i) au moins un composé à base d'urée provenant de la transformation d'au moins un isocyanate avec au moins une amine et (ii) au moins un polyéther comprenant des groupes hydroxyle bloqués, qui est exempt de groupes uréthane.

2. Agent thixotropique selon la revendication 1, **caractérisé en ce que** le composé à base d'urée présente la formule (I) suivante 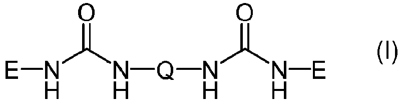 dans laquelle
E représente un radical alkyle ou cycloalkyle comprenant 1 à 12 atomes de carbone et
Q représente un radical hydrocarboné bivalent, comprenant 6 à 15 atomes de carbone.

3. Agent thixotropique selon la revendication 1 ou 2, **caractérisé en ce que** l'agent thixotropique se trouve sous forme d'une pâte stable, pouvant être enduite.

4. Agent thixotropique selon l'une quelconque des revendications 1 à 3, **caractérisé en ce qu'**il contient 5 à 25% en poids de composé à base d'urée et 50 à 95% en poids de polyéther comprenant des groupes hydroxyle bloqués.

5. Agent thixotropique selon l'une quelconque des revendications 1 à 4, **caractérisé en ce que** le polyéther comprenant des groupes hydroxyle bloqués présente une viscosité à 20°C dans la plage de 30 à 500 mPa.s, la viscosité étant déterminée à l'aide d'un viscosimètre à cône-plaques présentant un diamètre de cône de 25 mm, un angle de cône de 1°, une distance pointe de cône-plaques de 0,05 mm à une vitesse de cisaillement de 10 s⁻¹.

6. Agent thixotropique selon l'une quelconque des revendications 1 à 5, **caractérisé en ce que** le polyéther présente en moyenne 1 à 3 groupes hydroxyle bloqués par molécule.

7. Agent thixotropique selon l'une quelconque des revendications 1 à 6, **caractérisé en ce que** les groupes hydroxyle bloqués sont choisis dans le groupe constitué par les groupes acétate, ester, acétoester et carbonate.

8. Agent thixotropique selon l'une quelconque des revendications 1 à 7, **caractérisé en ce que**, dans le polyéther, 70 à 100% en poids des motifs récurrents sont constitués de groupes 1,2-propylénoxy et 0 à 30% en poids des motifs récurrents sont constitués de groupes 1,2-éthylénoxy.

9. Agent thixotropique selon l'une quelconque des revendications 1 à 8, **caractérisé en ce que** le polyéther comprenant des groupes hydroxyle bloqués présente un poids moléculaire moyen Mₙ dans la plage de 600 à 2500 g/mole, déterminé par chromatographie par perméation de gel (CPG) par rapport au polystyrène en tant que référence avec du tétrahydrofuranne en tant que phase mobile, un détecteur d'indice de réfraction et une interprétation à partir de 200 g/mole.

10. Agent thixotropique selon l'une quelconque des revendications 1 à 9, **caractérisé en ce que** le polyéther comprenant des groupes hydroxyle bloqués est dérivé d'au moins un polyéther à fonctionnalité hydroxy choisi dans le groupe constitué par
- les mono-ols de polyoxypropylène initiés par un alcool, en particulier le n-butanol, présentant un indice d'OH dans la plage de 25 à 90 mg de KOH/g,
- les diols de polyoxypropylène présentant un indice d'OH dans la plage de 45 à 155 mg de KOH/g et
- les triols de polyoxypropylène initiés par du triméthylolpropane ou en particulier du glycérol, le cas échéant terminés par oxyde d'éthylène, présentant une fonctionnalité OH moyenne dans la plage de 2,2 à 3 et un indice d'OH dans la plage de 90 à 230 mg de KOH/g.

11. Préparation de l'agent thixotropique selon l'une quelconque des revendications 1 à 10, **caractérisée en ce que** la transformation entre l'isocyanate et l'amine est réalisée dans le polyéther présentant des groupes hydroxyle bloqués.

12. Utilisation de l'agent thixotropique selon l'une quelconque des revendications 1 à 10 dans une composition durcissable en vue d'augmenter la limite d'écoulement de la composition.

13. Composition durcissable comprenant l'agent thixotropique selon l'une quelconque des revendications 1 à 10 et au moins un polymère contenant des groupes isocyanate et/ou silane.

14. Composition durcissable selon la revendication 13, **caractérisée en ce qu'**elle est à un composant et durcissable par l'humidité.

15. Article collé ou étanchéifié ou revêtu par la composition durcissable selon l'une quelconque des revendications 13 à 14.
